# EUROPEAN PATENT APPLICATION

(11) **EP 3 037 106 A1**
(43) Date of publication of application: **29.06.2016**
(21) Application number: 14307130.6
(22) Date of filing: 22.12.2014
(51) Int. Cl.: A61K 45/00, A61P 35/00

(54) **TXA2 inhibitor as stimulator or inducer of MHC Class I cross-presentation**

(71) Applicant: INSTITUT PASTEUR, 75724 Paris Cedex 15 (FR)
(72) Inventor: Bobard, Alexandre, 92100 BOULOGNE BILLANCOURT (FR); Enninga, Jost, 75005 PARIS (FR)
(74) Representative: Paris, Fabienne

(57) **Abstract**

The application relates to a thromboxane A2 (TXA₂) inhibitor. The application also relates to an Antigen Presenting Cell (APC), in the cytosol of which said TXA₂ inhibitor is contained. Said TXA₂ inhibitor can e.g., be a Non-Steroidal Anti-Inflammatory Drug (NSAID).

More particularly, the application relates to means, such as preparations, compositions, and methods, which comprise or involve said TXA₂ inhibitor or APC.

Said TXA₂ inhibitor or APC can notably be used as stimulator or inducer of the immune response, more particularly of MHC Class I cross-presentation.

## Description

### FIELD OF THE INVENTION

The application relates to a thromboxane A2 (TXA₂) inhibitor. The application also relates to an Antigen Presenting Cell (APC), which comprises or contains said TXA₂ inhibitor, more particularly to an APC, in the cytosol of which said TXA₂ inhibitor is contained. Said TXA₂ inhibitor can *e.g.,* be a Non-Steroidal Anti-Inflammatory Drug (NSAID).

More particularly, the application relates to means, such as preparations, compositions and methods, which comprise or involve said TXA₂ inhibitor or said APC.

Said TXA₂ inhibitor, APC or means can notably be used as stimulator or inducer of the immune response, more particularly of MHC Class I cross-presentation.

### BACKGROUND OF THE INVENTION

Cross-presentation is a crucial antigen presentation pathway driving the immune system to destroy tumor cells and pathogen infected cells (Fehres et al. 2014, Frontiers in Immunology, volume 5, article 149, 10 pages). Despite its importance in mammals, the cell biology of cross-presentation remains poorly understood. Cross-presenting cells need to transfer endosomal/phagosomal antigens towards the cytosol in order to be processed by the proteasome. Their further surface presentation triggers the cytotoxic CD8+ T cell response.

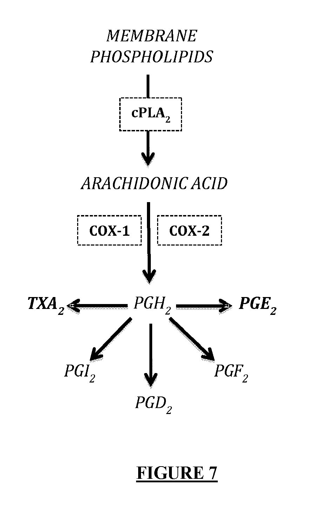

Early studies suggested the involvement of the Endoplasmic Reticulum Associated Degradation (ERAD) and the channel Sec61 as the *bona fide* cytosolic translocator for the antigen trafficking during cross presentation. These results were challenged by a number of other studies. In recent years, several research groups have attempted to isolate factors that participate in the cytosolic transfer of antigens using the processing and presentation of ovalbumin derived peptides as model, but none of them were shown to be directly involved in the translocation *per se.* Prostanoids are a family of lipid-derived molecules, which comprise prostaglandin and thromboxanes, and which are involved in a variety of cellular processes as diverse as inflammation, angiogenesis, thrombosis and immunosuppression. Prostanoid biosynthesis notably involve the COX enzymes (COX-1 and COX-2; *cf.* Figure 7), which can be inhibited by Non-Steroidal Anti-Inflammatory Drugs (NSAIDs).

Ahmadi et al. 2008 (Cancer Res. 68(18): 7520-7529) teaches that prostaglandin E₂ (PGE₂) should be inhibited to produce activation of antitumor CTL cell response.

Harris 2009 is a review of epidemiologic literature and reports that the use of NSAIDs as COX-2 inhibiting agents significantly reduced the risk of colon, breast, prostate and lung cancers (mainly using aspirin or ibuprofen as non-selective COX-2 inhibitors).

WO 2005/037995 in the name of CEDARS-SINAI MEDICAL CENTER relates to the use of a selective COX2 inhibitor in an amount sufficient to selectively inhibit prostaglandin E2 (PGE₂) in the treatment of COX2-overexpressing glioma. WO 2005/037995 teaches that the inhibition of PGE₂ allows glioma-infiltrating dendritic cells to polarize Th cells towards the Th1 subset.

However, NSAIDs are also described as immune-suppressive agents. For example, Iñiguez et al. 1999 (The Journal of Immunology 163: 111-119) teaches that the COX-2 inhibitors NS398 and celecoxib are immunosuppressive drugs.

The application provides means, which are especially adapted for inducing or stimulating MHC class I cross-presentation in an Antigen Presenting Cell (APC), more particularly in a macrophage or dendritic cell, more particularly in a macrophage. The means of the application notably involve the inhibition of the thromboxane A2 (TXA₂) biosynthesis pathway.

### SUMMARY OF THE INVENTION

The inventors demonstrate that the thromboxane A2 (TXA₂) biosynthesis pathway is involved in the transfer of antigen in the cytosol of Antigen Presenting Cell (APC), more particularly of dendritic cell and macrophage, more particularly of macrophage.

More particularly, the inventors demonstrate that the inhibition of the TXA₂ biosynthesis pathway leads to an induction or stimulation of said cytosolic transfer, whereby leading to an induction or stimulation of MHC class I cross-presentation in said APC.

Said TXA₂ inhibitor can *e.g.,* be a Non-Steroidal Anti-Inflammatory Drug (NSAID). Antigen transfer as well as cell surface cross-presentation in APC (such as macrophage) can be induced or drastically increased upon NSAID treatment.

The application relates to said TXA₂ inhibitor or to an APC (or APCs), which comprises or contains said TXA₂ inhibitor, more particularly to an APC, in the cytosol of which said TXA₂ inhibitor is contained.

The application also relates to means, such as preparations, compositions and methods, which comprise or involve said TXA₂ inhibitor or said APC(s).

Said TXA₂ inhibitor, APC(s) or means can notably be used as stimulator or inducer of the immune response, more particularly of MHC Class I cross-presentation.

### BRIEF DESCRIPTION OF THE FIGURES

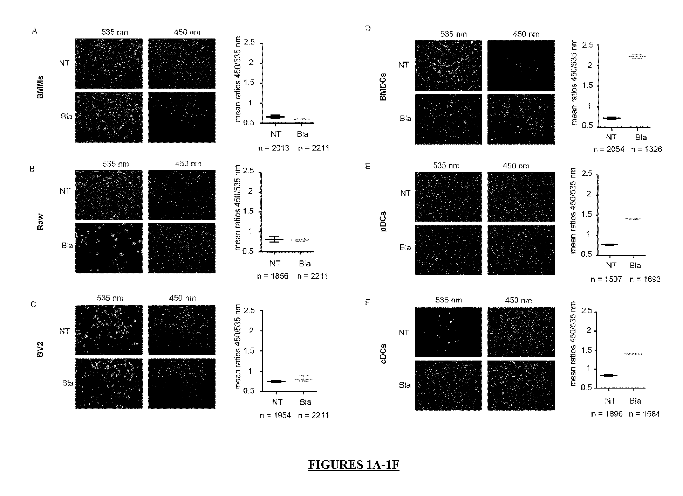

**Figures 1A-1F****:** Unlike macrophages, dendritic cells efficiently transfer β-lactamase from the endosomal compartment to the cytosol.
   BMMs (A), Raw macrophages (B), BV2 macrophages (C), BMDCs (D), pDCs (E) or cDCs (F) were incubated or not with 1 mg/ml β-lactamase at 37°C for 90 minutes after preloading them with CCF4-AM at 37°C for 1h. WIDEFIELD imaging was performed at 20X magnification using 535 and 450 nm emission filters and 405 nm excitation. The plots show the scored 450/535 nm average ratios for each condition. Numbers of measured cells are indicated for each condition, error bars indicate standard deviation.

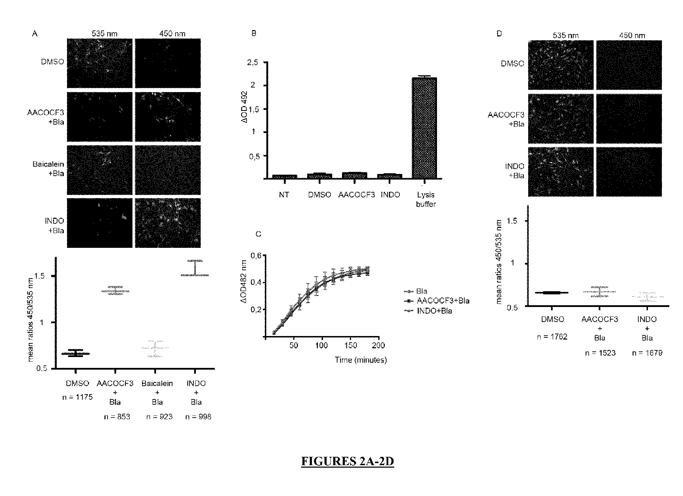

Figures 2A-2D: Blocking prostanoïd, but not leukotriene biosynthesis, trigger the transfer of β-lactamase from the endosomal compartment to the cytosol in bone marrow derived macrophages.
   BMMs were incubated for 15 minutes at 37°C with CCF4-AM added either with DMSO 1%, AACOCF3 0.25mM, baïcalein 1mM or indomethacin 1mM. Subsequently, cells were incubated or not with 1 mg/ml β-lactamase at 37°C (A) or 4°C (D) for 90 minutes, washed and imaged using NIKON TI WIDEFIELD microscope at 20X magnification with 535 and 450 nm filters. Average ratios for the whole population are visualized on a plot for each condition. Numbers of measured cells are indicated for each condition, error bars indicate standard deviation. (B) BMMs were loaded for 15 minutes at 37°C with CCF4-AM added either with DMSO 1%, AACOCF3 0.25mM or indomethacin 1mM. Subsequently, supernatants were subjected to a cytotoxicity detection test for assaying LDH release from damaged cells at OD492 nm. (C) After 15 minutes at 37°C with DMSO 1%, AACOCF3 0.25mM or indomethacin 1mM, BMMs were incubated or not with 1 mg/ml β-lactamase at 37°C for 15 minutes. After 3 washes and cell lysis, total intracellular β-lactamase activity is assayed in function of time using the colorimetric substrate nitrocefin as a read-out at OD 482 nm for 3 hours.

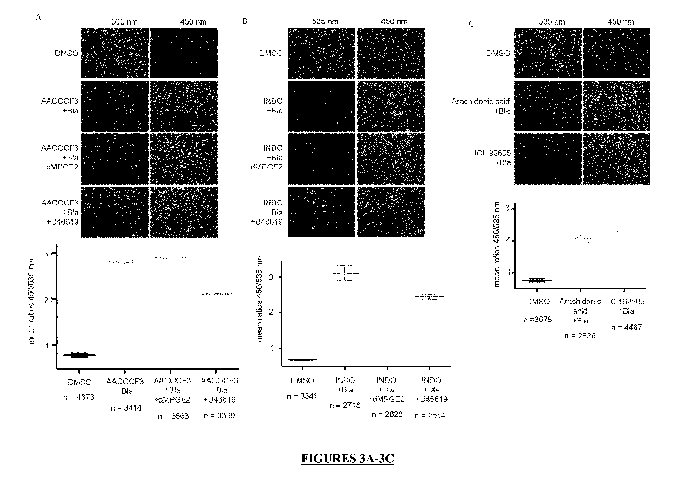

**Figures 3A-3C****:** Thromboxane A2 depletion and arachidonic acid accumulation are involved in the transfer of β-lactamase to the cytosol triggered upon COX and PLA2 inhibition in bone marrow derived macrophages.
   After 15 minutes at 37°C with CCF4-AM added either with DMSO 1%, AACOCF3 0.25mM (A), indomethacin 1mM (B), acide arachidonic 80µM or ICI192605 1mM (C), BMMs were incubated or not with 1 mg/ml β-lactamase added with or without diMePGE2 or U46619 10µM for 90 minutes at 37°C. Then, cells were washed and imaged using NIKON TI WIDEFIELD microscope at 20X magnification using 535 and 450 nm filters. The plot represents the average ratios of the cell population for each condition. Numbers of measured cells are indicated for each condition, error bars indicate standard deviation.

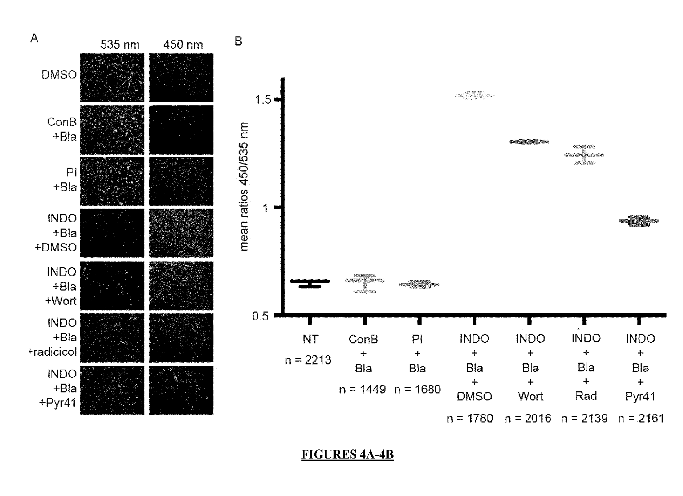

**Figures 4A-4B****:** Indomethacin induced transfer of β-lactamase to the cytosol of bone marrow derived macrophages is not solely a consequence of increased endosomal antigen survival and is restrained by wortmannin, radicicol and Pyr41.
   (A) Bone marrow derived macrophages were incubated for 15 minutes at 37°C with CCF4-AM added either with DMSO 1% or indomethacin 1mM. Subsequently, cells were incubated or not with 1 mg/ml β-lactamase added with or without concanamycin B 25nM, protease inhibitors 1X, wortmannin 0.5µM, radicicol 25µM or Pyr41 10µM for 90 minutes at 37°C. After washing, cells were imaged using NIKON TI WIDEFIELD microscope at 20X magnification with 535 and 450 nm filters. (B) Average ratios of the whole population were obtained and plotted for each condition. Numbers of measured cells are indicated for each condition, error bars indicate standard deviation.

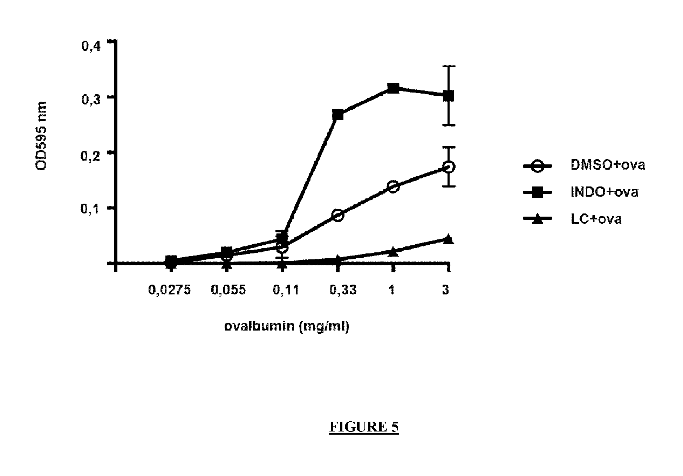

**Figure 5****:** Indomethacin promotes cross-presentation of ovalbumin in bone marrow derived dendritic cells
   After 15 minutes at 37°C with or without DMSO 1%, indomethacin 1mM or lactacystine 3µM, BMDCs were incubated with serial dilutions of ovalbumin for 4 hours at 37°C and put in contact with B3Z hybridoma cells at a ratio 1:1 overnight. After washing, intracellular lacZ activity is assayed using CPRG at OD 595 nm.

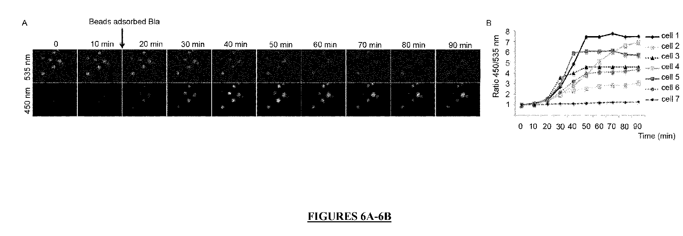

**Figures 6A-6B****:** The transfer of β-lactamase to the cytosol can be visualized in real-time in JAWS-II dendritic cells
   (A) JAWS-II dendritic cells were loaded for 1 hour with CCF4-AM and subjected to a 90 minutes time-lapse video-microscopy using NIKON TI WIDEFIELD microscope with 450 and 535 nm channels. Beads adsorbed with β-lactamase were added 10 minutes after starting the movie. Snapshots taken every 10 minutes are shown on the figure. (B) Ratios 450/535 nm are scored for each individual cells and represented as a plot in function of time.
**Figure 7****:** Overview of the prostanoïd biosynthesis pathway
   Under inflammatory conditions, cPLA2 activation induces the release of arachidonic acid from membrane phospholipid to the cytosol. The further conversion of arachidonic acid to PGH2 by COX-1 or COX-2 allows then the production of biologically active prostanoïds PGE2, PGF2, PGD2, PGI2 and TXA2.
**Figure 8****:** Indomethacin does not trigger the transfer of β-lactamase to the cytosol in HeLa cells Following HeLa cells incubation for 15 minutes at 37°C with CCF4-AM added either with DMSO 1% or indomethacin 1mM, 1 mg/ml β-lactamase was added or not at 37°C for 90 minutes. After washing, cells are imaged using NIKON TI WIDEFIELD microscope at 20X magnification with 535 and 450 nm filters and data are plotted together as mean ratios 450/535 nm for each condition.
**Figure 9****:** Sulindac triggers the transfer of β-lactamase to the cytosol in bone marrow macrophages
   BMMs were loaded for 15 minutes with CCF4-AM added with DMSO 1% or sulindac sulphide 1mM and were subsequently subjected to β-lactamase incubation for 90 minutes. After washing, cells are imaged using NIKON TI WIDEFIELD microscope at 20X magnification with 535 and 450 nm filters and data are plotted together as mean ratios 450/535 nm for each condition.

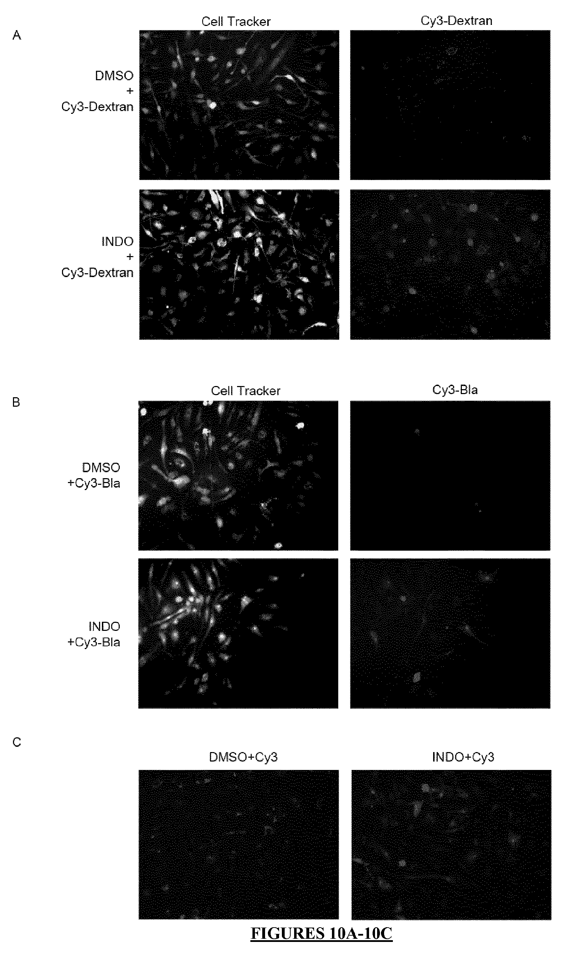

**Figure 10A-10C****:** Indomethacin triggers the transfer of Cy3 labeled β-lactamase, dextran and Cy3 to the cytosol in bone marrow derived macrophages.
   After 15 minutes DMSO 1% or indomethacin 1mM treatment, BMMs were incubated with 10-kDa Cy3-dextran 1 mg/ml (A), Cy3-Bla 0.2 mg/ml (B) or Cy3 0.2 mg/ml (C) for 90 minutes. Cells were then washed, labeled with CELLMASK and imaged using NIKON TI WIDEFIELD microscope at 20X magnification with 570 and 647 nm filters.

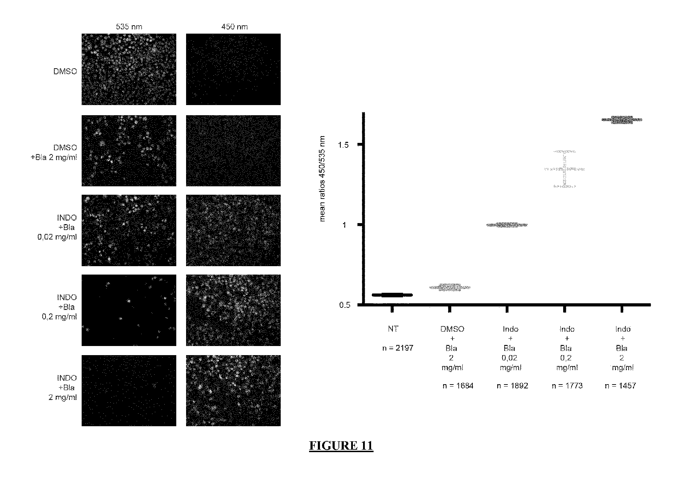

**Figure 11****:** Indomethacin induced cytosolic transfer depends on the external concentration of β-lactamase in BV2 macrophages-like cells.
   BV2 cells were loaded for 15 minutes at 37°C with CCF4-AM added either with DMSO 1% or indomethacin 1mM. Subsequently, cells were incubated or not with increasing amount of β-lactamase at 37°C for 90 minutes. Following washing, cells are imaged using NIKON TI WIDEFIELD microscope at 20X magnification using 535 and 450 nm filters. Scored 450/535 nm average ratios are then plotted for each condition.

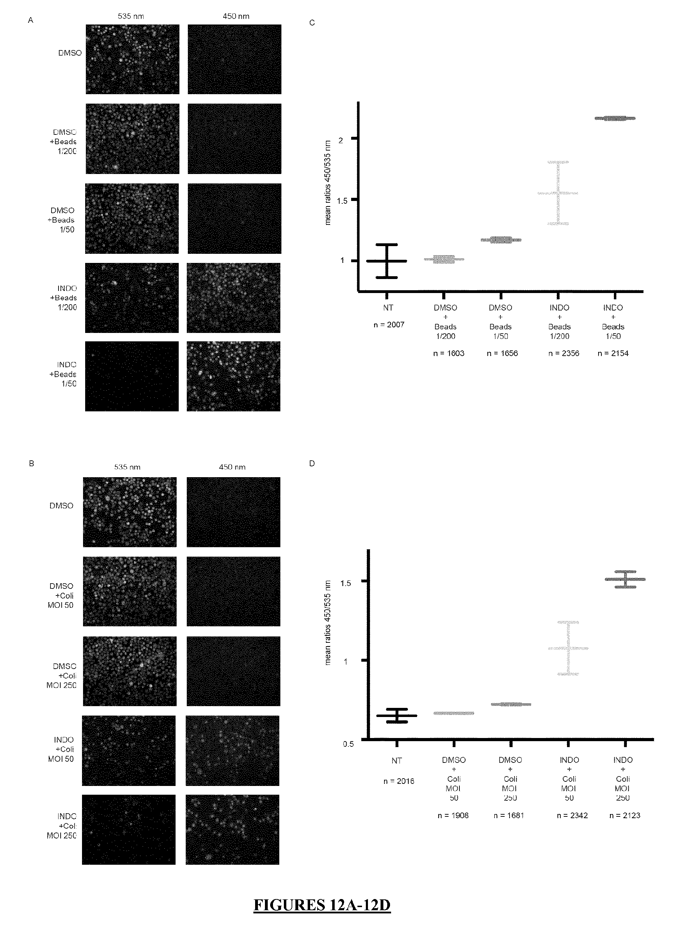

**Figures 12A-12D****:** Indomethacin is also able to trigger the transfer of β-lactamase when adsorbed to beads or expressed by *E. coli* in BV2 macrophages-like cells in a dose dependent manner.
   After 15 minutes loading at 37°C with CCF4-AM added either with DMSO 1% or indomethacin 1mM, cells were incubated or not with increasing amount of beads adsorbed (A, C) or *E. coli* expressed β -lactamase (B, D) at 37°C for 90 minutes. After washing, cells are imaged using NIKON TI WIDEFIELD microscope at 20X magnification with 535 and 450 nm filters. The plot represents the average ratios of the cell population for each condition.

### DETAILED DESCRIPTION OF THE INVENTION

The present application relates to the subject-matter as defined in the claims as filed and as herein described.

The application relates to at least one thromboxane A2 (TXA₂) inhibitor, and/or to at least one Antigen Presenting Cell (APC), which comprises or contains said TXA₂ inhibitor, more particularly to at least one APC, in the cytosol of which said (at least one) TXA₂ inhibitor is contained. Said TXA₂ inhibitor, or APC containing it, advantageously induces or stimulates an immune response, more particularly a CD8+ immune response, more particularly a Cytotoxic T Lymphocyte (CTL) immune response.

Said immune response is a response to at least one antigen, in a mammal, more particularly a human. Said at least one antigen can be an antigen, which has been administered to said non-human mammal or human (antigen derived from an infectious agent or a vaccine), and/or an antigen, which has not been administered to said mammal, but which is naturally produced by the body of said non-human mammal or human (antigens from self-tissues). More particularly, said non-human mammal or human is a diseased non-human mammal or human, *e.g*., a non-human mammal or human affected by a cancer or infectious disease.

More particularly, said TXA₂ inhibitor, or APC containing it, induces or stimulates MHC class I cross-presentation, more particularly MHC class I cross-presentation by APC, such as dendritic cells and/or macrophages, more particularly MHC class I cross-presentation by macrophages. MHC class I cross-presentation is intended in accordance with its ordinary meaning in the field. Schematically, MHC Class I cross-presentation refers to the presentation by an APC of an antigen, which is of exogenous origin (*i.e.,* which is extracellular and has been internalized by said APC, such as an antigen of tumor or microbial origin) through MHC class I molecules (Fehres et al. 2014, Frontiers in Immunology volume 5, article 149, 10 pages; Nierkens et al. 2013, Trends in Immunology 34(8): 361-370; Joffre et al. 2012, Nature Reviews Immunology 12: 557-569).

Hence, in accordance with an advantageous feature, said TXA₂ inhibitor or APC containing it induces or stimulates the priming of CTL by an APC, more particularly by a macrophage and/or a dendritic cell, more particularly by a macrophage.

In accordance with the application, said APC cross-presents said at least one antigen (*via* MHC class I).

This is in contrast to the direct priming of CTL by a pathogenic cell (*e.g.,* a tumor cell or a bacteria or virus infected cell), which would carry said at least one antigen.

The application thus relates to a thromboxane A2 (TXA₂) inhibitor (or an APC containing it, more particularly, a macrophage or dendritic cell containing it, more particularly a macrophage containing it) for use as a stimulator or inducer of MHC Class I cross-presentation of at least one antigen.

The application relates more particularly to said TXA₂ inhibitor or APC containing it for use as a stimulator or inducer of MHC Class I cross-presentation of at least one antigen in the treatment and/or prevention and/or palliation of a cancer or infectious disease in a mammal (more particularly a human), wherein said at least one antigen is a tumor antigen of said cancer or is an antigen of the infectious agent of said infectious disease respectively.

Said treatment and/or prevention and/or palliation comprises the administration of said TXA₂ inhibitor or APC containing it to inhibit, more particularly to selectively inhibit, TXA₂ activity and/or TXA₂ biosynthesis in an Antigen Presenting Cell (APC), in said mammal.

Said at least one antigen or an APC containing it may have been administered to said non-human mammal or human (e.g in a vaccine), *e.g*., to induce or stimulate an immune response against said at least one antigen, and/or may not have been administered to said non-human mammal or human, *e.g*., because said at least one antigen is produced by the body of said (diseased) non-human mammal or human naturally, *i.e.,* without human intervention (antigen from self-tissues). Said TXA₂ inhibitor or APC containing it can induce or stimulate said immune response. It is more particularly helpful when the immune response raised by said administered antigen is insufficient or deficient, for example due to anergy of immune cells, such as APCs, of said mammal or due to any other immune-inhibitory phenotype.

The administration of said TXA₂ inhibitor or APC containing it can be a local administration, more particularly a local administration at the site or primary site of said cancer or infectious disease. Such a local administration can *e.g.,* be intended for stimulating MHC class I cross-presentation by APCs (more particularly by dendritic cells and/or macrophages, more particularly by macrophages), which are located at said site or primary site, *e.g*., for stimulating MHC class I cross-presentation by APCs (more particularly by the dendritic cells and/or macrophages, more particularly by the macrophages), which have infiltrated said site or primary site (e.g., the site or primary site of said cancer or infection).

Alternatively or complementarily, the administration of said TXA₂ inhibitor or APC containing it can be a systemic administration, *e.g*., a parenteral administration.

The administration of said TXA₂ inhibitor or APC containing it can be simultaneous, separate or sequential to the administration of said at least antigen or to said APC containing it (exogenous administration of said at least one antigen or APC containing it). This is notably the case when the administration of said TXA₂ inhibitor or APC containing it is a systemic administration. Alternatively or complementarily, the administration of said TXA₂ inhibitor or APC containing it can be a "stand alone" administration, *i.e.,* an administration, which is not simultaneous, not separate and not sequential to the administration of any antigen or antigen-containing APC (endogenous presence of said at least one antigen or APC containing it). This is notably the case when the administration of said TXA₂ inhibitor or APC containing it is a local administration.

The application also relates to a preparation, which comprises the following two i. and ii. elements:
i. at least one antigen, and/or at least one APC, which comprises or contains said at least one antigen, more particularly to an APC, in the cytosol of which said at least one antigen is contained,
   and
ii. at least one thromboxane A2 (TXA₂) inhibitor, and/or at least one APC, which comprises or contains said at least one TXA₂ inhibitor, more particularly to at least one APC, in the cytosol of which said (at least one) TXA₂ inhibitor is contained.

Advantageously, said preparation is a combined preparation. More particularly, said at least one antigen or APC of i. and said at least one TXA₂ inhibitor or APC of ii. are in combination for simultaneous, separate or sequential administration to a mammal (e.g., a human), more particularly for simultaneous, separate or sequential administration to a mammal (e.g., a human) in need of MHC Class I cross-presentation of said at least one antigen.

When said at least one antigen or APC of i. and said at least one TXA₂ inhibitor or APC of ii. are in combination for simultaneous administration, said preparation is a composition, which comprises said at least one antigen or APC of i. and said at least one TXA₂ inhibitor or APC of ii., more particularly a composition, which comprises said at least one antigen or APC of i. and said at least one TXA₂ inhibitor or APC of ii. in mixture.

Said preparation, combined preparation or composition can be a pharmaceutical preparation, combined preparation or composition.

Said preparation, combined preparation or composition can be an immunogenic composition, combined preparation or composition.

Said preparation, combined preparation or composition can be a vaccine.

Advantageously, the preparation, combined preparation or composition of the application is suitable for administration to a mammal. For example, said at least one antigen or APC of i. and said at least one TXA₂ inhibitor or APC of ii. are each or both contained in a pharmaceutically acceptable vehicle or diluent, more particularly a pharmaceutically and/or physiologically acceptable vehicle or diluent, which is suitable for administration to a mammal.

The preparation, combined preparation or composition of the application can *e.g.,* be sterile or otherwise devoid of any enzyme.

Advantageously, said at least one antigen is an antigen of a pathogenic agent, which does not and/or cannot infect APCs of a mammal (the antigen is exogenous). Said mammal can be a non-human mammal or human. Advantageously, said mammal is a human.

The TXA₂ inhibitor of the application or the APC containing it, as well as the preparation, combined preparation or composition of the application are especially adapted to MHC Class I presentation of said at least one antigen.

Indeed, the inventors demonstrate that inhibiting TXA₂ induces or stimulates MHC class I cross-presentation, more particularly MHC Class I cross-presentation in an APC, more particularly MHC Class I cross-presentation in a dendritic cell or macrophage, more particularly MHC Class I cross-presentation by a macrophage.

Advantageously, said at least one TXA₂ inhibitor (or APC containing it) is for administration to a mammal, e.g., a human, in need of MHC Class I cross-presentation of said at least one antigen. Advantageously, said at least one antigen (or APC containing it) is in or administered to a mammal, *e.g*., a human, in need of MHC Class I cross-presentation of said at least one antigen.

More particularly, said TXA₂ inhibitor is contained in said preparation, combined preparation or composition, or is administered to said mammal, in an amount sufficient to and/or at a site of the body appropriate to inhibit, more particularly to selectively inhibit, TXA₂ activity and/or TXA₂ biosynthesis in an Antigen Presenting Cell (APC), which is contained in said mammal, and in the cytosol of which said at least one antigen is contained.

More particularly, said TXA₂ inhibitor is contained in said preparation, combined preparation or composition, or is administered to said mammal, in an amount sufficient to and/or at a site of the body appropriate to stimulate the MHC Class I cross-presentation of said at least one antigen by said APC, which is contained in said mammal, and in the cytosol of which said at least one antigen is contained.

Advantageously, said APC, which contains said at least one antigen, is a dendritic cell or a macrophage, more particularly a macrophage.

In the preparation, combined preparation or composition of the application, said at least one APC of i. and/or said at least one APC of ii. advantageously is(are) a dendritic cell or a macrophage, more particularly a macrophage.

Said at least one APC of i. can be of the same APC type as said at least one APC of ii. For example, each of said at least one APC of i. and/or said at least one APC of ii. is a dendritic cell. For example, each of said at least one APC of i. and/or said at least one APC of ii. is a macrophage.

Said at least one APC of i. can be the same cell as said at least one APC of ii. For example, the at least one antigen of i. and the at least one TXA₂ inhibitor of ii. are both contained in the cytosol of the same APC, more particularly of the same dendritic cell or macrophage, more particularly of the same macrophage.

Thromboxane A2 (TXA₂) is the compound of formula C₂₀H₃₂O₅ shown below:

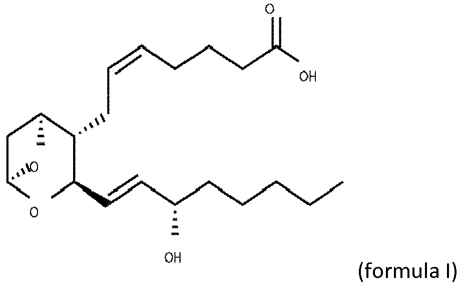

TXA₂ is described in the PUBCHEM^{®} data base under accession number CID 5280497.

Said at least one TXA₂ inhibitor can *e.g.,* be a compound, which binds to:
- TXA₂, and/or to
- the TXA₂ receptor, and/or to
- one or several of the molecules or complexes that contributes to TXA₂ biosynthesis.

Said at least one TXA₂ inhibitor can *e.g.,* be a TXA₂ ligand and/or an antagonist of the TXA₂ receptor and/or an inhibitor of TXA₂ biosynthesis, more particularly an antagonist of the TXA₂ receptor and/or an inhibitor of TXA₂ biosynthesis.

For example, said at least one TXA₂ inhibitor is a TXA₂ ligand, or an antagonist of the TXA₂ receptor, or an inhibitor of TXA₂ biosynthesis, more particularly, an antagonist of the TXA₂ receptor or an inhibitor of TXA₂ biosynthesis.

Said TXA₂ ligand can *e.g.,* be a compound, which binds to TXA₂ and inhibits the binding of TAX₂ to its receptor, *i.e.,* the TXA₂ receptor. More particularly, said TXA₂ ligand is a compound, which can be delivered or which can enter in the cytosol of an APC, more particularly of a dendritic cell and/or macrophage, more particularly of a macrophage.

Said antagonist of the TXA₂ receptor can *e.g.,* be a compound, which binds to the TXA₂ receptor and inhibits the binding or activation of TXA₂ to the TXA₂ receptor.

The thromboxane A2 (TXA₂) receptor is also known as the prostanoid TP receptor (Hirata et al. 1991, Nature 349: 617-620; Raychowdhury et al. 1994, The Journal of Biological Chemistry 269(30): 19256-19261; Narumiya et al. 1999 Physiological Reviews 70(4): 1193-1226). The human TXA₂ receptor is encoded by the TBXA2R gene.

The reference human cDNA sequence for the TXA₂ receptor sequence is available under accession number NM_001060:

1 cccacccgtc ccagctcggc ttagcccccg cggacccctc caggccgcga ccccagggcg
61 tcctgtgccg ccacgccctc catctgtgtg ggtcctctgc tgggcccgcc cctggtcaca
121 gccagactga ctcagtttcc ctgggaggtc ccgctcgagc ccgtccttcc cctccctctg
181 cccgccccca gccctcgccc caccctcggc gcccgcacat ctgcctgctc agctccagac
241 ggcgcccgga cccccgggcg cgggatccag ccaggtggga gccccgcaga tgaggtctct
301 gaaggtgtgc ctgaaccagt gccagcctgc cctgtctgca gcatcggcct gatggggtgg
361 tgactgatcc ctcagggctc cggagccatg tggcccaacg gcagttccct ggggccctgt
421 ttccggccca caaacattac cctggaggag agacggctga tcgcctcgcc ctggttcgcc
481 gcctccttct gcgtggtggg cctggcctcc aacctgctgg ccctgagcgt gctggcgggc
541 gcgcggcagg ggggttcgca cacgcgctcc tccttcctca ccttcctctg cggcctcgtc
601 ctcaccgact tcctggggct gctggtgacc ggtaccatcg tggtgtccca gcacgccgcg
661 ctcttcgagt ggcacgccgt ggaccctggc tgccgtctct gtcgcttcat gggcgtcgtc
721 atgatcttct tcggcctgtc cccgctgctg ctgggggccg ccatggcctc agagcgctac
781 ctgggtatca cccggccctt ctcgcgcccg gcggtcgcct cgcagcgccg cgcctgggcc
841 accgtggggc tggtgtgggc ggccgcgctg gcgctgggcc tgctgcccct gctgggcgtg
901 ggtcgctaca ccgtgcaata cccggggtcc tggtgcttcc tgacgctggg cgccgagtcc
961 ggggacgtgg ccttcgggct gctcttctcc atgctgggcg gcctctcggt cggctgtcc
1021 ttcctgctga acacggtcag cgtggccacc ctgtgccacg tctaccacgg gcaggaggcg
1081 gcccagcagc gtccccggga ctccgaggtg gagatgatgg ctcagctcct ggggatcatg
1141 gtggtggcca gcgtgtgttg gctgcccctt ctggtcttca tcgcccagac agtgctgcga
1201 aacccgcctg ccatgagccc cgccgggcag ctgtcccgca ccacggagaa ggagctgctc

1261 atctacttgc gcgtggccac ctggaaccag atcctggacc cctgggtgta tatcctgttc
1321 cgccgcgccg tgctccggcg tctccagcct cgcctcagca cccggcccag gtcgctgtcc
1381 ctccagcccc agctcacgca gcgctccggg ctgcagtagg aagtggacag agcgcccctc
1441 ccgcgccttt ccgcggagcc cttggcccct cggacagccc atctgcctgt tctgaggatt
1501 caggggctgg gggtgctgga tggacagtgg gcatcagcag caggttttgg ggttgacccc
1561 aatccaaccc ggggaccccc aactcctccc tgatcctttt accaagcact ctcccttcct
1621 cggccccttt ttcccatcca gagctcccac cccttctctg cgtccctccc aaccccagga
1681 agggcatgca gacattggaa gagggtcttg cattgctatt ttttttttta gacggagtct
1741 tgctctgtcc cccaggctgg agtgcagtgg cgcaatctca gctcactgca acctccacct
1801 cccgggttca agcgattctc ctgcctcagc ctcctgagta gctgggacta taggcgcgcg
1861 ccaccacgcc cggctaattt ttgtattttt agtagagacg gggtttcacc gtgttggcca
1921 ggctggtctt gaactcctga cctcaggtga ttcaccagcc tcagcctccc aaagtgctgg
1981 gatcacaggc atgaaccacc acacctggcc attttttttt ttttttttag acggagtctc
2041 actctgtggc ccagcctgga gtacagtggc acgatctcgg ctcactgcaa cctccgcctc
2101 ccgggttcaa gcgattctcg tgcctcagcc tcccgagcag ctgggattac aggcgtaagc
2161 cactgcgccc ggccttgcat gctctttgac cctgaatttg acctacttgc tggggtacag
2221 ttgcttcctt ttgaacctcc aacaggaaag gctctgtcca gaaaggattg aatgtgaacg
2281 ggggcacccc cttttcttgc caaaatatat ctctgccttt ggttttattt tcctttgggt
2341 ccagaagttt tcaatctctg gagtttgtgc attgggcctc acccggagca gacgagggca
2401 gggctgggaa ggatggggag ggacatagag gattcggttc ctcccccacc ttgtttcttg
2461 agtgtttcct aatcacttga tgggtggtac cacctagaat tcttccagca gcctgtaatg
2521 ggagggctac aggttagccc tggaacctgc aatcaaacca cctttgaatc tgtgtgtcat
2581 taaaagtaga tataaatggg caaaaaaaaa aaaaaaaaa SEQ ID
NO: 1

In humans, two TXA₂ receptor splice variants, TPₐₗₚₕₐ and TP_{beta}, have been cloned.

The reference sequence of the human TXA₂ receptor isoform alpha is available under accession number NP_001051:

1 
61 
121 
181 
241 
301 
SEQ ID NO: 2

The reference sequence of the human TXA₂ receptor isoform beta is available under accession number NP_963998 :

1 mwpngsslgp cfrptnitle errliaspwf aasfcvvgla snllalsvla garqggshtr
61 ssfltflcgl vltdflgllv tgtivvsqha alfewhavdp gcrlcrfmgv vmiffglspl
121 llgaamaser ylgitrpfsr pavasqrraw atvglvwaaa lalgllpllg vgrytvqypg
181 swcfltlgae sgdvafgllf smlgglsvgl sfllntvsva tlchvyhgqe aaqqrprdse
241 vemmaqllgi mvvasvcwlp llvfiaqtvl rnppamspag qlsrttekel liylrvatwn

301 qildpwvyil frravlrrlq prlstrprrs ltlwpsleys gtisahcnlr lpgssdsras

361 asraagitgv shcarpcmlf dpefdllagv qllpfepptg kalsrkd SEQ ID NO: 3

In the application, an inhibitor or antagonist of the (human) TXA2 receptor is meant as an inhibitor or antagonist of at least one of isoform alpha and isoform beta of said receptor, more particularly of each of the two isoforms.

An inhibitor of TXA₂ biosynthesis can *e.g.,* be an inhibitor of an enzyme of the TXA₂ biosynthesis pathway, more particularly an inhibitor of TXA₂ synthase and/or of cyclooxygenase-2 (COX-2) and/or of cyclooxygenase (COX) and/or of phospholipase A2 (PLA₂), still more particularly an inhibitor of TXA₂ synthase or of COX-2 or of PLA₂, still more particularly an inhibitor of TXA₂ synthase or of COX-2, still more particularly more particularly an inhibitor of TXA₂ synthase.

A reference human cDNA sequence for TXA₂ synthase is available under accession number NM_001061.

A reference human cDNA sequence for COX-2 is available under accession number NM_00963. COX refers to COX 1 and COX-2; a reference human cDNA sequence for COX-1 is available under accession number NM_080591.

A reference description of PLA₂ is available under enzyme accession number EC 3.1.1.4.

Said at least one TXA₂ inhibitor can *e.g.,* be a Non-Steroidal Anti-Inflammatory Drug (NSAID) or a pharmaceutically acceptable salt thereof.

Examples of NSAID, which are antagonists of the TXA₂ receptor, comprise:
**ICI-192605** (4-(Z)-6-(2-o-Chlorophenyl-4-o-hydroxyphenyl-1,3-dioxan-cis-5-yl)hexenoic acid), SQ-29548 ([1S-[1α,2α(Z),3α,4α]]-7-[3-[[2-[(phenylamino)carbonyl]hydrazino]methyl]-7-oxabicyclo[2.2.1]hept-2-yl]-5-heptenoic acid),
**terutroban** (S18886) (3-[(6R)-6-(4-chlorobenzenesulfonamido)-2-methyl-5,6,7,8-tetrahydronaphthalen-1-yl]propanoic acid),
**ifetroban** (BMS 180291) (3-[2-[[(1S,2R,3S)-3-[4-(pentylcarbamoyl)-1,3-oxazol-2-yl]-7-oxabicyclo[2.2.1]heptan-2-yl]methyl]phenyl]propanoic acid),
**seratrodast** (AA-2414) (7-(3,5,6-Trimethyl-1,4-benzoquinon-2-yl)-7-phenylheptanoic acid),
**solutroban** (BM-13177) (2-[4-[2-(benzenesulfonamido)ethyl]phenoxy]acetic acid),
SQ-28668 ((Z)-7-[(1S,2R,3S,4R)-3-[(E)-3-hydroxy-4-phenylpent-1-enyl]-7-oxabicyclo[2.2.1]heptan-2-yl]hept-5-enoic acid),
**ONO-3708** ((Z)-7-[(1R,3S,4S,5S)-3-[[(2R)-2-cyclopentyl-2-hydroxyacetyl]amino]-6,6-dimethyl-4-bicyclo[3.1.1]heptanyl]hept-5-enoic acid),
**ramatroban** (Bay U3405) (3R-[[(4-fluorophenyl)sulfonyl]amino]-1,2,3,4-tetrahydro-9H-carbazole-9-propanoic acid),
**EP-045** ((Z)-7-[(1R,2R,3R,4R)-3-[(E)-(phenylcarbamoylhydrazinylidene)methyl]-2-bicyclo[2.2.1]heptanyl]hept-5-enoic acid),
**domitroban** (S-145) ((Z)-7-[(1R,2S,3S,4S)-3-(benzenesulfonamido)-2-bicyclo[2.2.1]heptanyl]hept-5-enoic acid),
**picotamide** (which also is a TXA₂ synthase inhibitor) (4-methoxy-1-N,3-N-bis(pyridin-3-ylmethyl)benzene-1,3-dicarboxamide), and
the pharmaceutically acceptable salts thereof.

Examples of NSAID, which are inhibitors of TXA₂ synthase, comprise:
**OKY-046** (3-[4-(1H-imidazol-1-ylmethyl)phenyl]-2E-propenoic acid),
**picotamide** (which also is a TXA₂ receptor antagonist) (4-methoxy-1-N,3-N-bis(pyridin-3-ylmethyl)benzene-1,3-dicarboxamide), and
the pharmaceutically acceptable salts thereof.

Examples of NSAID, which are inhibitors of COX-2, comprise:
**rofecoxib** (MK-966) (4-(4-methylsulfonylphenyl)-3-phenyl-5H-furan-2-one),
**meloxicam** (4-hydroxy-2-methyl-N-(5-methyl-2-thiazolyl)-1,1-dioxide-2H-1,2-benzothiazine-3-carboxamide),
**celecoxib** (SC 58635) (4-[5-(4-methylphenyl)-3-trifluoromethyl)pyrazol-1-yl]benzenesulfonamide)
**diclofenac** (2-[(2,6-dichlorophenyl)amino]-benzeneacetic acid),
**sulindac** (5-fluoro-2-methyl-1Z-[[4-(methylsulfinyl)phenyl]methylene]-1H-indene-3-acetic acid) or a pharmaceutically acceptable salt thereof,
**piroxicam** (4-hydroxy-2-methyl-N-2-pyridinyl-2H-1,2-benzothiazine-3-carboxamide-1,1-dioxide),
**parecoxib** (N-{[4-(5-methyl-3-phenylisoxazol-4-yl)phenyl]sulfonyl}propanamide),
**etoricoxib** (5-chloro-6'-methyl-3-[4-(methylsulfonyl)phenyl]-2,3'-bipyridine),
**valdecoxib** (4-(5-methyl-3-phenylisoxazol-4-yl)benzenesulfunamide),
**lumiracoxib** ({2-[2-chloro-6-fluorophenyl)amino]-5-methylphenyl}acetic acid),
**firocoxib** (3-(cyclopropylmethoxy)5,5-dimethyl-4-(4-methylsulfonylphenyl)furan-2-one),
**NS-398** (N-[2-(cyclohexyloxy)-4-nitrophenyl]-methanesulfonamide),
**DuP-697** (5-bromo-2-(4-fluorophenyl)-3-(4-(methylsulfonyl)phenyl)-thiophene), and
the pharmaceutically acceptable salts thereof.

Examples of NSAID, which are inhibitors of COX, comprise:
**ibuprofen** (α-methyl-4-(2-methylpropyl)-benzeneacetic acid),
**naproxen** (6-methoxy-α-methyl-2-naphthaleneacetic acid),
**aspirin** (2-(acetyloxy)-benzoic acid),
**indomethacin** (1-(4-chlorobenzoyl)-5-methoxy-2-methyl-1H-indole-3-acetic acid) and
the pharmaceutically acceptable salts thereof.

Examples of NSAID, which are inhibitors of PLA₂, comprise:
**AACOCF3** (1,1,1-trifluoro-6Z,9Z,12Z,15Z-heneicosatetraen-2-one) and
the pharmaceutically acceptable salts thereof.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit leukotriene biosynthesis.

Said at least one TXA₂ inhibitor can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase and/or of COX-2, more particularly an inhibitor of TXA₂ synthase or of COX-2, more particularly an inhibitor of TXA₂ synthase,
which does not inhibit leukotriene biosynthesis.

An inhibitor, which does not inhibit leukotriene biosynthesis, is *e.g.,* an inhibitor, which does inhibit lysyl oxidase (LOX). A reference human cDNA sequence for LOX is available under accession number NM_002619.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit prostaglandin E2 (PGE2) biosynthesis.

PGE2 is described in the PUBCHEM^{®} data base under accession number CID 5280360.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase,
which does not inhibit PGE2 biosynthesis.

Advantageously, said at least one TXA₂ inhibitor or NSAID is:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit leukotriene biosynthesis, and which does not inhibit prostaglandin E2 (PGE2) biosynthesis.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase,
which does not inhibit leukotriene biosynthesis, and which does not inhibit PGE₂ biosynthesis.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit the biosynthesis of prostaglandins E₂, F₂ and D₂ (PGE₂, PGF₂ and PGD₂, respectively).

PGF₂ is described in the PUBCHEM^{®} data base under accession number CID 5280363.

PGD₂ (alpha) is described in the PUBCHEM^{®} data base under accession number CID 448457.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase,
which does not inhibit prostaglandin the biosynthesis of PGE₂, PGF₂ and PGD₂.

Advantageously, said at least one TXA₂ inhibitor or NSAID is:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit leukotriene biosynthesis, and which does not inhibit the biosynthesis of PGE₂, PGF₂ and PGD₂.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase,
which does not inhibit leukotriene biosynthesis, and which does not inhibit the biosynthesis of PGE₂, PGF₂ and PGD₂.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit the biosynthesis of PGE₂, PGF₂ and PGD₂, and of prostacyclin *i.e.,* of prostaglandin I₂ (PGI₂).

PGI₂ is described in the PUBCHEM^{®} data base under accession number CID 5282411.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase,
which does not inhibit prostaglandin the biosynthesis of PGE₂, PGF₂, PGD₂ and PGI₂.

Advantageously, said at least one TXA₂ inhibitor or NSAID is:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit leukotriene biosynthesis, and which does not inhibit the biosynthesis of PGE₂, PGF₂, PGD₂ and PGI₂.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase,
which does not inhibit leukotriene biosynthesis, and which does not inhibit the biosynthesis of PGE₂, PGF₂, PGD₂ and PGI₂.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit cyclooxygenase-1 (COX-1).

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase and/or of COX-2, more particularly an inhibitor of TXA₂ synthase or of COX-2, more particularly an inhibitor of TXA₂ synthase,
which does not inhibit COX-1.

Advantageously, said at least one TXA₂ inhibitor or NSAID is:
- an inhibitory ligand of TXA₂ or an antagonist of the TXA₂ receptor, more particularly an antagonist of the TXA₂ receptor,
   and/or (more particularly, or)
- an inhibitor of TXA₂ biosynthesis,
which does not inhibit leukotriene biosynthesis, and which does not inhibit COX-1.

Said at least one TXA₂ inhibitor or NSAID can *e.g.,* be:
- an inhibitory ligand of TXA₂ or an antagonist of the TXA₂ receptor, more particularly an antagonist of the TXA₂ receptor,
   and/or (more particularly, or)
- an inhibitor of TXA₂ synthase,
which does not inhibit leukotriene biosynthesis, and which does not inhibit COX-1.

Said TXA₂ inhibitory ligand can *e.g.,* be a compound, more particularly a NSAID, which selectively or specifically binds to TXA₂ [and inhibits its binding to the TXA₂ receptor]. For example, a compound, which selectively binds to TXA₂, has a half maximal effective concentration (EC₅₀) for TXA₂ that is lower than its EC₅₀ for other prostanoid(s), more particularly for PGE₂, more particularly for PGE₂, PGF₂, PGD₂ and PGI₂.

Said antagonist of the TXA₂ receptor can *e.g.,* be a compound, more particularly a NSAID, which is a non-competitive or competitive antagonist of the TXA₂ receptor.

A non-competitive antagonist binds to an allosteric site of the receptor, or irreversibly binds to the active site of the receptor [and inhibits the binding or activation of the TXA₂ receptor by TXA₂].

A competitive antagonist reversibly binds to the receptor at the same binding site as the endogenous ligand (TXA₂) [and inhibits the binding or activation of the TXA₂ receptor by TXA₂].

Said antagonist of the TXA₂ receptor can *e.g.,* be a compound, more particularly a NSAID, which selectively inhibits said TXA₂ receptor compared to at least one of the PGE₂, PGF₂, PGD₂ and PGI₂ receptors, more particularly for at least the PGE2 receptor, more particularly for all of the PGE₂, PGF₂, PGD₂ and PGI₂ receptors.

The human PGE₂ receptor is encoded by human gene PTGER2 (reference cDNA sequence of accession number NM_000956).

The human PGF₂ receptor is encoded by human gene PTGFR (reference cDNA sequence of accession number NM_000959).

The human PGD₂ receptor is encoded by human gene PTGDR (reference cDNA sequence of accession number NM_000953).

The human PGI₂ receptor is encoded by human gene PTGIR (reference cDNA sequence of accession number NM_000960).

For example, said antagonist of the TXA₂ receptor can *e.g.,* be a compound, more particularly a NSAID, which has an EC50 for the TXA₂ receptor that is lower than its EC50 for at least one of the PGE₂, PGF₂, PGD₂ and PGI₂ receptors, more particularly for at least the PGE2 receptor, more particularly for all of the PGE₂, PGF₂, PGD₂ and PGI₂ receptors.

Said antagonist of the TXA₂ receptor can *e.g.,* be a compound, more particularly a NSAID, which specifically inhibits the TXA₂ receptor.

Said inhibitor of TXA₂ biosynthesis can *e.g.,* be a compound, more particularly a NSAID, which is a non-competitive (or competitive) inhibitor of one or several enzyme(s), which contributes or are involves in TXA₂ biosynthesis (such as PLA₂, COX, COX-2 and TXA₂ synthase).

More particularly, said inhibitor of TXA₂ biosynthesis can *e.g*., be a compound, more particularly a NSAID, which is a non-competitive inhibitor of said enzyme(s).

A non-competitive inhibitor of an enzyme is a ligand of the complex formed by said enzyme and the substrate of said enzyme. For example, a non-competitive inhibitor of TXA₂ biosynthesis can *e.g.,* be a compound, more particularly a NSAID, which is:
- a ligand of the complex formed by TXA₂ synthase and prostaglandin H2 (PGH2), and/or (more particularly, or)
- a ligand of the complex formed by COX-2 and arachidonic acid, and/or (more particularly, or)
- a ligand of the complex formed by COX and arachidonic acid, and/or (more particularly, or)
- a ligand of the complex formed by PLA2 and phospholipids.

A competitive inhibitor of an enzyme is a ligand of said enzyme. For example, a competitive inhibitor of TXA₂ biosynthesis can *e.g.,* be a compound, more particularly a NSAID, which is:
- a ligand of TXA₂ synthase, and/or (more particularly, or)
- a ligand of COX-2, and/or (more particularly, or)
- a ligand of COX, and/or (more particularly, or)
- a ligand of PLA₂.

Said inhibitor of TXA₂ biosynthesis can *e.g.,* be a selective TXA₂ synthase inhibitor, a selective COX-2 inhibitor, or a selective COX inhibitor, a selective PLA₂ inhibitor.

Said inhibitor of TXA₂ biosynthesis can *e.g.,* be a compound, more particularly a NSAID, which selectively inhibits the enzyme(s) to which it binds, more particularly which selectively inhibits at least one of TXA₂ synthase, COX-2, COX and PLA₂.

Said inhibitor of TXA₂ biosynthesis can *e.g.,* be a compound, more particularly a NSAID, which selectively inhibits TXA₂ synthase or COX-2 or COX or PLA₂, more particularly TXA₂ synthase.

For example, said inhibitor of TXA₂ biosynthesis can *e.g*., be a compound, more particularly a NSAID, which has an EC50 for at least one of TXA₂ synthase, COX-2, COX and PLA₂, more particularly for one of TXA₂ synthase, COX-2, COX and PLA₂, more particularly for TXA₂ synthase, which is lower than its EC50 for LOX.

Said inhibitor of TXA₂ biosynthesis can *e.g.,* be a compound, more particularly a NSAID, which selectively inhibits TXA₂ synthase compared to other prostaglandin synthase(s), such as PGE₂, PGF₂, PGD₂ and PGI₂ synthases.

More particularly, said inhibitor of TXA₂ biosynthesis can *e.g*., be a compound, more particularly a NSAID, which has a EC50 for TXA₂ synthase that is lower than its EC50 for at least one of PGE₂, PGF₂, PGD₂ and PGI₂ synthases, more particularly for at least the PGE₂ synthase, more particularly for all the PGE₂, PGF₂, PGD₂ and PGI₂ synthases.

Said inhibitor of TXA₂ biosynthesis can *e.g.,* be a compound, more particularly a NSAID, which selectively inhibits COX-2 compared to COX-1.

For example, said inhibitor of TXA₂ biosynthesis can *e.g*., be a compound, more particularly a NSAID, which has an EC50 for COX-2 that is lower than its EC50 for COX-1.

Said inhibitor of TXA₂ biosynthesis can *e.g.,* be a compound, more particularly a NSAID, which selectively inhibits COX or COX-2 compared to LOX.

For example, said inhibitor of TXA₂ biosynthesis can *e.g.*, be a compound, more particularly a NSAID, which has an EC50 for COX or for COX-2 that is lower than its EC50 for LOX.

Said inhibitor of TXA₂ biosynthesis can *e.g.,* be a compound, more particularly a NSAID, which selectively inhibits PLA₂ compared to phospholipases A1, C and D (PLA₁, PLC and PLD, respectively).

For example, said inhibitor of TXA₂ biosynthesis can *e.g*., be a compound, more particularly a NSAID, which has an EC50 for PLA₂ that is lower than its EC50 for PLA₁, PLC and PLD.

Said inhibitor of TXA₂ biosynthesis can *e.g.,* be a compound, more particularly a NSAID, which specifically inhibits TXA₂ synthase or COX-2 or COX or PLA₂, more particularly which specifically inhibits TXA₂ synthase.

Said at least one antigen can *e.g.,* be a peptide antigen, more particularly a peptide antigen of 8-11 amino acids, for example a peptide antigen of 8, 9, 10 or 11 amino acids, more particularly of 9, 10 or 11 amino acids. Hence, said at least one antigen can *e.g.,* be a peptide antigen of 8-11 amino acids, which is an antigen of a pathogenic agent, which does not and/or cannot infect an APC of said mammal (exogenous antigen).

Said pathogenic agent can *e.g.,* be a tumor, *i.e.,* said at least one antigen can be a tumor antigen. Said tumor antigen can *e.g.,* be:
- a lung tumor antigen (e.g., a lung carcinoma antigen, a lung squamous cell carcinoma antigen),
- a head and neck squamous cell carcinoma antigen,
- a brain tumor antigen, a pancreas tumor antigen,
- a melanoma antigen,
- a leukemia antigen.

Said tumor antigen can *e.g.,* be a Tumor-Specific Antigen (TSA) or a Tumor-Associated Antigen (TAA), more particularly a TSA.

Tumor-Specific Antigens (TSA) are antigens, which are expressed in tumors, but which are (generally) not expressed in normal tissues (except maybe by some specific normal cells such as placental cells and testicular germ cells).

Tumor-Associated Antigens (TAA) are antigens, which are expressed in tumors, as well as in normal tissue, more particularly in the normal tissue of origin of the malignancy.

Examples of tumor antigens, more particularly of TSA, comprise the antigens of SEQ ID NOs: 1-6 shown in Table 1 below.

**Table 1:**

| Gene/protein | Tumor | Peptide | SEQ ID NO: |
|---|---|---|---|
| alpha-actinin-4 | lung carcinoma | FIASNGVKLV | 4 |
| Elongation factor 2 | lung squamous cell carcinoma | ETVSEQSNV | 5 |
| NFYC | lung squamous cell carcinoma | QQITKTEV | 6 |
| ME1 | non-small cell lung carcinoma | FLDEFMEGV | 7 |
| CASP-8 | head and neck squamous cell carcinoma | FPSDSWCYF | 8 |
| p53 | head and neck squamous cell carcinoma | VVPCEPPEV | 9 |

Said tumor antigen can *e.g.,* be selected from SEQ ID NOs: 1-6. Said tumor antigen can *e.g.,* be SEQ ID NO: 4 or NO: 5 or NO: 6 or NO: 8 or NO: 8 or NO: 9.

Said at least one antigen can *e.g.,* be an antigen of an infectious agent, more particularly a microbial antigen, more particularly a bacterial or viral antigen.

Hence, said at least one antigen can *e.g.,* be an antigen of a microorganism, more particularly of a bacterium or virus, which does not and/or cannot infect an APC (exogenous antigen) of said mammal.

More particularly, said microorganism, bacterium or virus is a pathogenic microorganism, bacterium or virus.

Said bacterium can *e.g.,* be *Mycobacterium, Nocardia, Legionella, Chlamydia, Salmonella, Yersinia* or *Coxiella,* more particularly *Mycobacterium.*

Said *Mycobacterium* can *e.g.,* be M. *tuberculosis, M. bovis* or M. *avium,* more particularly M. *tuberculosis.*

Said *Nocardia* can *e.g.,* be *Nocardia asteroids.*

Said *Legionella* can *e.g.,* be *Legionella pneumophila.*

Said *Chlamydia* can *e.g.,* be *Chlamydia trachomatis.*

Said *Salmonella* can *e.g.,* be *Salmonella enterica,* more particularly S. *enterica* serovar Typhimurium, or *S. enterica* serovar Typhi.

Said *Yersinia* can *e.g.,* be *Yersinia enterocolitica.*

Said *Coxiella* can *e.g.,* be *Coxiella burnetti.*

Said at least one antigen can be a soluble, i.e., a cell-free antigen, more particularly a synthetic cell-free antigen. Alternatively, said at least one antigen can be contained in a nanoparticle or in a liposome.

Said at least one antigen can be a monomer of the antigen sequence, or a multimer, more particularly a dendrimer of the antigen sequence. Said monomer, multimer or dendrimer can be glycosylated.

Said at least one antigen can *e.g.,* be linked, conjugated or fused to at least one protein or polypeptide or polysaccharide (e.g., at least one antibody, antibody fragment or glycan), which binds or specifically binds to an APC, more particularly to the APC cell type (e.g., macrophage. Examples of such proteins or polypeptides comprise antibodies or antibody fragments that are specific for the uptake receptor(s) of said APC, or glycans, which interact with C-type Lectin Receptors (CLRs) of said APC.

Said at least one antigen can *e.g.,* be linked, conjugated or fused to a CD74 (endolysosomal) targeting sequence, more particularly to a protein or polypeptide (e.g., an antibody or antibody fragment), which specifically binds to CD74, more particularly to human CD74 (the reference protein sequence of human CD74 is available under accession number NP_001020329; sequences of human CD74 isoforms are also available under accession numbers XP_006714874.1; NP_001020330.1; NP_001020329.1 and NP_004346.1).

Said (combined) preparation or composition can have any pharmaceutically and/or physiologically acceptable form. More particularly, said (combined) preparation or composition can have a form suitable for local administration (more particularly, for local administration at the site or primary site of said cancer or infectious disease) or for mucosal administration, or for systemic administration, more particularly for parenteral administration, *e.g*., for intravenous injection and/or for intraperitoneal injection and/or for subcutaneous injection and/or for transdermal injection and/or for transmuscular injection, more particularly for intravenous injection.

Said (combined) preparation or composition can *e.g.,* be a liquid solution, an emulsion, a suspension.

It can be formulated with any pharmaceutically and/or physiologically acceptable vehicle or diluent that is suitable for administration to a mammal, more particularly for injection into a mammal.

Appropriate pharmaceutically acceptable vehicles and formulations include all known pharmaceutically acceptable vehicles and formulations, such as those described in "Remington: The Science and Practice of Pharmacy", 20th edition, Mack Publishing Co.; and "Pharmaceutical Dosage Forms and Drug Delivery Systems", Ansel, Popovich and Allen Jr., Lippincott Williams and Wilkins.

For instance, parenteral formulations usually comprise a physiologically acceptable fluid, such as sterile water, sterile physiological saline, sterile balanced salt solution or a sterile buffer.

Said (combined) preparation or composition may contain pharmaceutical adjunct materials such as, for example, a pharmaceutically acceptable salt to adjust the osmotic pressure and/or a preservative.

Said TXA₂ inhibitor can *e.g.,* be contained in said preparation, combined preparation or composition, or can *e.g.,* be administered to said mammal, in an amount sufficient to and/or at a site of the body appropriate to stimulate MHC Class I cross-presentation of said at least one antigen by APCs, and which have infiltrated the site or primary site of said cancer or infectious disease in said mammal.

Said TXA₂ inhibitor of the application or APC containing it, as well as said (combined) preparation or composition of the application can be for use in immuno-prevention, immuno-therapy or immuno-palliation.

Advantageously, said immuno-prevention, immuno-therapy or immuno-palliation is the immuno-prevention, immuno-therapy or immuno-palliation of a disease caused by a pathogenic agent, which does and/or cannot infect an APC of said mammal (exogenous antigen).

Said TXA₂ inhibitor of the application or APC containing it, as well as said (combined) preparation or composition can be for use in the immuno-prevention, immuno-therapy or immuno-palliation of a cancer or of an infectious disease, or for use as an inducer or stimulator of the immune response in the prevention or treatment or palliation of a cancer or of an infectious disease.

Said cancer can *e.g.* be a cancer as above-described.

More particularly, said cancer can be a non-hematopoietic cancer, *i.e.,* a cancer, which can be treated and/or palliated and/or prevented by local administration of TXA₂ inhibitor of the application (or APC containing it), or of said (combined) preparation or composition.

More particularly, said cancer is not a cancer of the Central Nervous System, more particularly not a glioma.

Said cancer can *e.g.* be a cancer of a tissue of said mammal, *e.g.,* of an epithelium or of a connective tissue of said mammal, for example a carcinoma or sarcoma.

Said cancer can *e.g.* be a cancer which does not overexpress COX2.

Said infectious disease can *e.g.,* be an infectious disease involving a microorganism, which does not and/or cannot infect an APC of said mammal.

As described above, said microorganism can *e.g.,* be a virus or bacterium, for example a bacterium as above-described.

Accordingly, said (combined) preparation or composition may further comprise:
- at least one therapeutic agent suitable for or intended for the prevention, treatment or palliation of said cancer, more particularly at least one chemotherapeutic agent, or at least one targeted therapeutic agent (*e.g*., a monoclonal antibody), or at least one hormonal agent, suitable for or intended for the prevention, treatment or palliation of said cancer, or
- at least one antibiotic, more particularly at least one antibiotic suitable for or intended for the prevention, treatment or palliation of said infectious disease.

The application also relates to a kit, which comprises at least one (combined) preparation or composition of the application, and said at least one therapeutic agent or said at least one antibiotic.

Advantageously, said TXA₂ inhibitor of the application or APC containing it, as well as said (combined) preparation or composition can be for use to treat a defect in immune response, more particularly in anti-tumor immune response or in anti-infection immune response.

Said immune response advantageously is a CD8+ response, more particularly a CTL response.

Hence, said TXA₂ inhibitor of the application or APC containing it, as well as said (combined) preparation or composition can be for use as an inducer or stimulator of a CD8+ response, more particularly of a Cytotoxic T Lymphocytes (CTL) response, more particularly of a Cytotoxic T Lymphocytes (CTL) response that is (specifically) directed against said at least one antigen.

Advantageously, said TXA₂ inhibitor of the application or APC containing it, as well as said (combined) preparation or composition induces or stimulates the priming (or activation) of CTL by APC of said mammal, more particularly by macrophages and/or dendritic cells of said mammal, more particularly by macrophages of said mammal. In accordance with the application, said APC (cross-)present said at least one antigen. The CTL that are primed (or activated) by said APC are (specifically) directed against said at least one antigen.

Hence, said TXA₂ inhibitor of the application or APC containing it, as well as said (combined) preparation or composition can be for use in the prevention, treatment or palliation of a defect or insufficiency in antigen presentation, more particularly a defect or insufficiency in antigen cross-presentation, more particularly a defect or insufficiency in MHC class 1 cross-presentation. Said defect or insufficiency can more particularly be a defect or insufficiency in antigen presentation by APC, more particularly by macrophages and/or dendritic cells, more particularly by macrophages.

The TXA₂ inhibitor of the application or APC containing it, as well as said (combined) preparation or composition can thus advantageously be intended for those patients, who have such a defect or insufficiency, more particularly a defect or insufficiency in antigen presentation by APC, more particularly by macrophages and/or dendritic cells, more particularly by macrophages.

The TXA₂ inhibitor of the application or APC containing it, as well as said (combined) preparation or composition can thus advantageously be intended for those patients, who have a defect or insufficiency in MHC class I cross-presentation, more particularly those patients, whose APC ((at least some of their APC), more particularly macrophages and/or dendritic cells (at least some of their macrophages and/or dendritic cells), more particularly whose macrophages (at least some of their macrophages), have a defect in MHC class I cross-presentation.

The application also relates to a method of production of a drug, which comprises providing (or producing) at least one antigen or APC containing it as above-described, which further comprises providing (or producing) at least one TXA₂ inhibitor or APC containing it as above-described, and which further comprises combining said at least one antigen or APC containing it and said at least one TXA₂ inhibitor or APC containing it in a preparation, more particularly in a preparation for simultaneous, sequential or separate use.

Said drug is useful in immuno-prevention, immuno-therapy or immuno-palliation, as above-described.

The application also relates to a method of prevention, treatment or palliation in a mammal in need thereof, more particularly in a human in need thereof.

The method comprises administering to said mammal the (combined) preparation or composition as above-described. More particularly, said (combined) preparation or composition is administered in an amount sufficient to and/or at a site of the body appropriate induce or stimulate the priming of CTL by APC of said mammal, more particularly by macrophages and/or dendritic cells of said mammal, more particularly by macrophages of said mammal.

Said method of prevention, treatment or palliation can *e.g.,* be a method of vaccination.

In the application, unless specified otherwise or unless a context dictates otherwise, all the terms have their ordinary meaning in the relevant field(s).

The term "comprising", which is synonymous with "including" or "containing", is open-ended, and does not exclude additional, unrecited element(s), ingredient(s) or method step(s), whereas the term "consisting of" is a closed term, which excludes any additional element, step, or ingredient which is not explicitly recited.

The term "essentially consisting of" is a partially open term, which does not exclude additional, unrecited element(s), step(s), or ingredient(s), as long as these additional element(s), step(s) or ingredient(s) do not materially affect the basic and novel properties of the invention.

The term "comprising" (or "comprise(s)") hence includes the term "consisting of" ("consist(s) of"), as well as the term "essentially consisting of" ("essentially consist(s) of"). Accordingly, the term "comprising" (or "comprise(s)") is, in the present application, meant as more particularly encompassing the term "consisting of" ("consist(s) of"), and the term "essentially consisting of" ("essentially consist(s) of").

In an attempt to help the reader of the present application, the description has been separated in various paragraphs or sections. These separations should not be considered as disconnecting the substance of a paragraph or section from the substance of another paragraph or section. To the contrary, the present description encompasses all the combinations of the various sections, paragraphs and sentences that can be contemplated.

Each of the relevant disclosures of all references cited herein is specifically incorporated by reference. The following examples are offered by way of illustration, and not by way of limitation.

### EXAMPLES

Cancer surveillance and intracellular pathogen elimination is mediated by T lymphocytes. To achieve this, specialized cells have to engulf and process tumor or microbial derived antigens in a cellular pathway called cross-presentation. Surface presentation of antigens for the activation of T cells requires their transfer from endomembrane compartment to the cytosol. Although crucial the mechanism underlying this step remains unknown.

In vivo, it is commonly thought that dendritic cells are the main cross-presenting antigen presenting cells (APCs), at least in mice. Nevertheless, other cell types, such as macrophages and neutrophils have also been proposed. Three antigen processing pathways have been suggested in the context of cross-presentation. The first one involves trafficking whereby extracellular antigens are transferred from the endosomal/phagosomal compartment to the cytosol for proteasomal processing and subsequent loading on the major histocompatibility complex class I (MHC I). Subsequently, peptide presentation at the cell surface triggers a cluster of differentiation 8+ (CD8+) T cell cytotoxic response. A second "vacuolar" pathway suggests antigen processing in endomembrane compartments, and a third pathway involves direct cell-to-cell transfer of peptides through gap junctions. Among them, the cytoplasmic pathway has emerged as most relevant since proteasome inhibition blunts CD8+T cell activation.

Early studies suggested the involvement of the Endoplasmic Reticulum Associated Degradation (ERAD) and the channel Sec61 as the *bona fide* cytosolic translocator for the antigen trafficking during cross presentation. These results were challenged by a number of other studies. In recent years, several research groups have attempted to isolate factors that participate in the cytosolic transfer of antigens using the processing and presentation of ovalbumin derived peptides as model, but none of them were shown to be directly involved in the translocation *per se.* This includes the molecular chaperone heat shock protein 90 (HSP90), the Soluble N-ethylmaleimide-sensitive-factor Attachment protein REceptor (SNARE) Sec22B and the endocytic receptor mannose receptor that respectively impact on antigen cytosolic refolding, antigen half-life and antigen internalization. The ESCRT protein TSG101 and the p97 AAA ATPase can also be added to this list potentially regulating mannose receptor ubiquitination and driving the transfer process. Studies on the mechanism of antigen transfer have been hampered, since methods for their study yield mostly crude readouts such as fluorescent antigen tracking, immunofluorescence and membrane fractionation. In order to circumvent these issues, we have developed a single cell based methodology to monitor cytoplasmic access of intact antigen before degradation by the proteasome using β-lactamase as a model antigen. Its principle relies on the loading of cells with the β-lactamase substrate CCF4-AM that accumulates in the cytosol and is excluded from intracellular endomembrane compartments. While this probe elicits Förster resonance energy transfer (FRET) at 535 nm (green) upon excitation at 405 nm, FRET is lost when β-lactamase reaches the cytoplasm cleaving the substrate and switching the emission to 450 nm (blue). Eicosanoids are lipid-derived molecules that are involved in a variety of cellular processes as diverse as inflammation, angiogenesis, thrombosis and immunosuppression. A major difference with pro-inflammatory cytokines is their very short half-life, restricting their role as autocrine and paracrine factors. Arachidonic acid produced through phospholipase A2 (PLA₂) from membrane phospholipids is the common backbone for the production of leukotrienes and prostanoïds respectively through the lipoxygenase (LOX) and the cyclooxygenase (COX) pathways. Two isoforms of COX have been characterized, one constitutive (COX-1) and another one inducible (COX-2).

Prostanoïds are crucial immunomodulators in the context of cancer progression, and increased levels of COX-2 in gastro-intestinal cancers represent a bad prognosis marker. Furthermore, the overproduction of prostaglandin E2 (PGE₂) by tumor cells was taught to prevent Tumor Infiltration Lymphocytes (TILs) (Ahmadi et al. 2008, Cancer Res 68(18): 7520-7529). Among all prostanoïds produced following COX induction, PGE₂ but also Thromboxane A2 (TXA₂) secreted by tumor cells and APCs display a clear pro-tumoral signature, while at the same time inhibiting apoptosis and CD8+ T cell infiltration.

Since cross-presentation is highly relevant for tumor surveillance, we decided to investigate the potential involvement of eicosanoids on this process focusing on the step of antigen cytosolic transfer and presentation. We adapted the CCF4/β-lactamase assay for the study of cytosolic antigen transfer via β-lactamase delivery *in vitro* in different living murine APCs cells at the single cell level confirming that dendritic cells have the unique capacity to perform this task efficiently. Screening for factors that could boost cross-presentation in APCs, we discovered a striking involvement of the prostanoïd pathway in this process with particularly strong effects in macrophages.

Indeed, blocking the axis PLA₂/COX/TXA₂ using chemical inhibitors forced the transfer of beta-lactamase to the cytosol without affecting antigen internalization, stability and turnover. This induced antigen transfer required the involvement of the ubiquitination machinery, the HSP90 molecular chaperone and the lipid kinase phosphatidylinositol 3-kinase (PI3K). Finally, we showed that blocking prostanoïd biosynthesis promoted cross-presentation of ovalbumin in dendritic cells. Together, this underlines the importance of prostanoïds in regulating not only the cytosolic delivery from endosomes but also the presentation of antigens at the plasma membrane.

### MATERIAL AND METHODS

**Primary Cells and Mice** - Bone marrow derived dendritic cells and macrophages were isolated by flushing femur and tibia from 6 to 9 weeks old C57/black-6 mice with ROSWELL PARK MEMORIAL INSTITUTE-1640 (RPMI-1640) supplemented with GLUTAMAX using 5 ml syringes (BD PLASTIPAK) and 0.5 x 16 mm needles 25G 5/8 (BD MicroLance). After centrifugation at 1000 rpm for 4 minutes, cells were resuspended in 1.66% NH₄Cl, and put at 4°C for 10 minutes. After centrifugation at 1,000 rpm for 4 minutes, cells were quantified and incubated at 37°C/5% CO₂ for 7 to 10 days in RPMI-1640 with GLUTAMAX containing 10% v/v fetal calf serum (GIBCO) and 50µg/ml penicillin/streptomycin (GIBCO). Granulocytes and macrophages-colony stimulating factor (GM-CSF, MILTENIY BIOTEC) and macrophages-colony stimulating factor (M-CSF, PREPROTECH) were added at day 0 at a concentration of 50 ng/ml in order to allow dendritic cells or macrophages differentiation. Dendritic cells were replated every 3 days adding each time fresh GM-CSF. Macrophages were supplemented with fresh M-CSF at day 4 and replated at day 7 with M-CSF at 25ng/ml. Plasmacytoïd and conventional dendritic cells were isolated from C57/black-6 mice spleen using a plasmacytoïd dendritic cell isolation kit (MILTENYI BIOTEC) and a CD8+ dendritic cell isolation kit (MILTENYI BIOTEC). BMDCs, pDCs and cDCs were adhered on 0.01% poly-lysine coated plates in phosphate buffer saline (PBS).

**Cell lines** - Murine Raw and BV2 macrophages like cells were cultured in RPMI-1640 with GLUTAMAX (GIBCO) containing 10% v/v fetal calf serum (GIBCO) and 50 µg/ml penicillin/streptomycin (GIBCO). B3Z were cultured in the same medium but supplemented with 1mg/ml G418 (SIGMA) and 0.4 mg/ml hygromycin B (SIGMA). JAWS-II cells were cultured in Iscove's Modified Dulbecco's Media (IMDM) with L-Glutamine and 25mM HEPES containing 10% v/v fetal calf serum (GIBCO), 50 µg/ml penicillin/streptomycin (GIBCO) and 5ng/ml GM-CSF. All cell lines were maintained at 37°C and 5% CO2.

**Reagents** - AACOCF3, indomethacin, U46619 and baïcalein were purchased at TOCRIS. Sulindac sulphide, probenecid, ICI192605, arachidonic acid and β-lactamase were purchased at SIGMA, and dimethyl-prostaglandin E2 at CALBIOCHEM. Red fluorescent 10kDa dextran was purchased at LIFE TECHNOLOGIES.

**Preparation of beads and bacteria** - *E. coli* DH5α were used expressing β-lactamase from a pBAD18 plasmid. After PBS washing, bacteria were re-suspended in EM buffer and the MOI was calibrated by measuring the absorbance of the overnight culture at OD 600nm. 3µm polystyrene beads (TEBU-BIO) were washed once before adsorption with β-lactamase at 4°C on a rotating wheel overnight. After PBS washing, beads were re-suspended in PBS buffer.

**FRET assay for antigen cytosolic transfer** - 30,000 to 50,000 cells were seeded on 96 well plates and loaded in a mix containing 3.3µM CCF4-AM (INVITROGEN), 1mM probenecid (SIGMA) and 1.5µl solution B (INVITROGEN) in EM buffer (120mM NaCl, 7mM KCI, 1.8mM CaCl2, 0.8mM MgCl2, 5mM glucose and 25mM HEPES at pH 7.3) in a final volume of 30 µl at 37°C. Following 1 mg/ml β-lactamase incubation in EM buffer, cells were washed with 1mM PBS/probenecid and imaged in EM buffer containing 1mM probenecid.

**Fluorescence microscopy and image analysis** - All acquisitions were performed on a NIKON TI WIDEFIELD microscope with a 20X N-Plan Objective. Experiments in triplicates were repeated at least 3 times independently for each condition using 25 pictures per replicate randomly and automatically. Image analysis was performed using custom-designed algorithms as described in Ray et al. 2010 (Cell Microbiol. 12(4): 545-556) and Simeone et al. 2012 (PLoS Pathog. 8(2): e1002507). Briefly, the 450 and 535 nm emission signals were scored automatically for each individual cells on a large set of images using METAMORPH. The quantitative parameters were then exported as an EXCEL file in which the ratio between the intensity of the 450 and 535 nm emission signals were presented as an average of the ratio in the whole cell population. After algorithm processing, mean ratios 450/535 nm from different replicates were obtained and pooled together as an average and the status of cytosolic transfer was assessed. Error bars represent standard deviation.

**Live cell imaging** - Live cell imaging was performed on an inverted NIKON TI microscope equipped with a heating chamber (37°C) using a 40X N-Plan Objective and a COOLSNAP CCD camera (ROEPER SCIENTIFIC). Cells were seeded in 96 well plates in EM buffer. Subsequently, beads with adsorbed β-lactamase were added at the start of time-lapse series. 450/535 ratios nm were analyzed using ImageJ as described in Ray et al. 2010 (Cell Microbiol. 12(4): 545-556). **Antigen uptake experiments** - A nitocefin activity test was used in order to assess the uptake level of β-lactamase under different conditions. This chromogenic β-lactamase substrate undergoes distinctive color change from yellow (390 nm) to red (486 nm) upon hydrolysis. Experiments were performed with 600,000 cells. After DMSO or compound treatment for 15 minutes, cells were incubated with 1 mg/ml β-lactamase at 37°C for 15 minutes, washed 3 times and lysed on ice using RIPA buffer (Sigma) and protein concentration was assessed (MICRO BCA Protein Assay Kit, THERMO SCIENTIFIC). Then, 1 µg of protein lysate was subjected to 0.1 mM nitrocefin at room temperature for 3 hours. OD 482 nm was assessed every 15 minutes using a FLUOstar OMEGA plate reader (BMG LABTECH). 3 independent experiments were performed in triplicate and plotted by GRAPHPAD. Error bars represent standard deviations.

Cytotoxicity experiments -Cytotoxicity was measured through LDH activity released from damaged cells using 30,000 cells per condition (ROCHE). Before, they were treated with DMSO or specific compounds in 40µl of EM buffer for 15 minutes. Then, 30 µl of the supernatants were subjected to the LDH detection kit following the instructions (ROCHE). Increased levels of OD 492 reflect plasma membrane damages, and were measured with a FLUOstar OMEGA plate reader

(BMG LABTECH). Three independent experiments in triplicates were analyzed by GRAPHPAD PRISM software (error bars represent standard deviations).

**B3Z cross-priming experiments** - 50,000 BMDCs per well were seeded on poly-L-lysine coated 96 well plates. After treatment with DMSO, 3µM lactacystine, or 1mM indomethacin for 15 minutes in RPMI-1640 complemented with 10% v/v fetal calf serum and 50 µg/ml penicillin/streptomycin at 37°C, cells were incubated with serial dilutions (from 3 to 0.0275 mg/ml) of ovalbumin (EUROMEDEX) for 4 hours. Triplicates were performed for each concentration and serial dilutions of the SIINFEKL 257-264 peptide (POLYPEPTIDE GROUP) were used as positive control (dilutions 1/10 starting from 0.1µg/ml). After 3 washes, cells are incubated overnight with 50,000 B3Z hybridoma cells at 37°C, washed once and subjected to the chlorophenol red-D-galactopyranoside (CPRG) detergent mix containing 100mM β-mercaptoethanol, 100mM MgCl₂, 0.125% NP40 and 0.15mM CPRG at 37°C for 4 hours. OD 492 as indicator of β-galactosidase (lacZ) activity produced under the control of the interleukin 2 promoter was subsequently measured by plate reader. Data were obtained from 3 independent experiments and plotted by GRAPHPAD PRISM (error bars: standard deviation).

### RESULTS

### A sensitive assay to track the delivery of antigen from endosomes to the cytosol in single APCs in real time

Aiming at the development of tools that monitor the intracellular trafficking of antigens during cross-presentation, we have adapted a sensitive single-cell based FRET assay for the measurements of cytosolic antigen transfer in living dendritic cells. *In vivo,* dendritic cells are the only cell type able to strongly trigger a CD8+ T cell based response. Thus, these cells should display a specific ability for targeting extracellular antigens to the cytosol following their uptake. We decided to test this hypothesis using our FRET assay with the model antigen β-lactamase on different APC models comparing the intracellular antigen trafficking route in macrophages and dendritic cells. Phagocytic cells efficiently internalize particles and molecules from the extracellular medium, as they constantly scan their environment in vivo for antigen detection. After loading different types of macrophages and dendritic cells with the cytosolic CCF4 FRET probe, we incubated them with β-lactamase for 90 minutes and analyzed the status of the FRET probe by WIDEFIELD fluorescence microscopy. Upon image acquisition, individual cells were automatically segmented, ratiometric analysis of fluorescence intensities at 450 and 535 nm was performed on thousands of cells per condition, and they were sampled into an average ratio in the whole cell population reflecting an intact FRET probe at low levels and a cleaved one at high levels (plots in Figures 1A-1F). In bone marrow derived macrophages (BMMs) (Figure 1A), but also in Raw (Figure 1B) and BV2 (Figure 1C) macrophage-like cell lines the CCF4 probe remained intact, emitting mostly in the green channel. Therefore, macrophages do not transfer the full-length β-lactamase into the cytosol efficiently as highlighted by the plots representing unchanged low average 450/535 ratios, despite their capacity to internalize up to 200% of their surface area every hour. In contrast, bone marrow derived dendritic cells (BMDCs) (Figure 1D) but also primary plasmacytoid DCs (pDCs) (Figure 1E) and conventional DCs (cDCs) (Figure 1F) displayed cytosolic transfer as shown by the cleavage of the CCF4 probe emitting in the blue channel after adding the antigen. Upon β-lactamase incubation, the average 450/535 nm ratio in the cell population doubled or even tripled in comparison to the control due to the cytosolic cleavage of the FRET probe. This validated our assay showing that the cytosolic CCF4 probe is well excluded *in situ* from antigen containing endosomal compartments and is not directly cleaved from the outside by extracellular antigen that leaks into the cells.

Furthermore, cytosolic transfer of the antigen was visualized in real time as shown by time-lapse imaging (Figure 6A) using the dendritic cell line JAWS-II with beads displaying adsorbed beta-lactamase. Tracking the 450/535 nm ratio from individual cells (Figure 6B), we found that the transfer process to the cytosol starts 10 minutes following β-lactamase incubation. Moreover, it took 30 to 80 min to fully cleave the FRET probe after adding exogenous antigens.

Thus, our single-cell based approach showed that macrophages and dendritic cells differentially process endocytosed antigens confirming that dendritic cells have an intrinsic capacity to target exogenous antigens efficiently to the cytosol. Furthermore, we revealed that this transfer occurs within the first hour of antigen exposure. This prompted us to address the striking differences between the different APCs in more detail asking why macrophages are less efficient in this task.

### PLA₂ and COX inhibition induce the cytosolic transfer of β-lactamase in macrophages

Cancer onset and progression is often associated with a local and chronic inflammatory state sustained by the tumor itself, but also by infiltrated leukocytes producing pro-inflammatory mediators. Among them, prostanoïds are phospholipid-derived molecules broken down from the plasma membrane under the action of PLA₂ (Figure 7) that promote tumor growth and dissemination. Since it is not known whether these lipid mediators directly affect the cell biology of cross-presentation during antigen trafficking and processing by APCs, we decided to use our FRET assay to test the impact of prostanoïd biosynthesis block on the delivery of antigens from endomembrane-bound compartments to the cytosol. Since dendritic cells manifest an intrinsic capacity to transfer ingested antigens to the cytosol, we rather tested the importance of eicosanoid depletion on the ability of cytosolic transfer in macrophages.

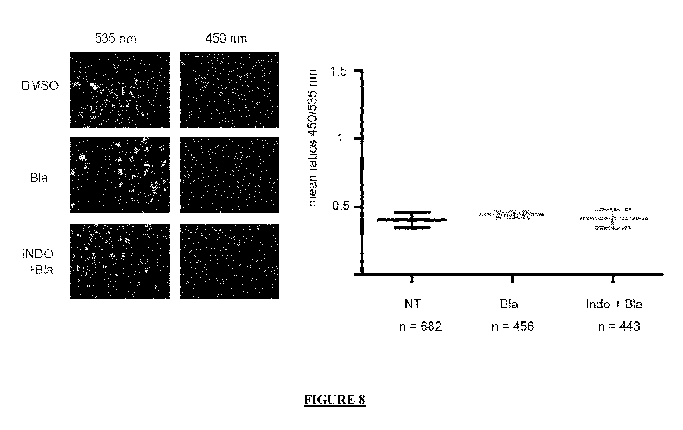

Our data showed that specific inhibition of PLA2 using arachidonyl trifluoromethyl ketone (AACOCF3) for 15 minutes followed by antigen incubation for 90 minutes at 37°C dramatically and robustly boosted transfer of the model antigen β-lactamase to the cytosol inside BMMs (Figure 2A). This implies that interfering with prostanoïd or leukotriene biosynthesis through the downstream regulators COX and LOX, should trigger the same transfer process. Interestingly, inhibiting COX but not LOX for 15 minutes using respectively indomethacin and baïcalein, led to a robust cytosolic transfer of beta-lactamase pointing at the implication of prostanoïds rather than leukotrienes in this process (Figure 2A). To rule out the possibility of direct antigen transfer from the extracellular milieu to the cytosol through the drug treatment without internalization we performed lactate deshydrogenase (LDH) release assays (Figure 2B). It only leaks out from cells after plasma membrane damage, for example during necrosis. We observed LDH release from BMMs upon compound addition was similar to the values in cells treated with dimethylsulfoxide (DMSO) underlining that the β-lactamase cytosolic delivery measured with our assay was not due to non-specific plasma membrane damage through the treatment of the used inhibitors (Figure 2B). As additional control, we also monitored plasma membrane integrity of HeLa cells treated with indomethacin showing no cytosolic β-lactamase delivery. This pointed at antigen transfer mechanism of our model antigen from an endomembrane compartment (Figure 8). During our experiments we controlled intracellular β-lactamase enzymatic activities as function of time using nitrocefin cleavage as a read-out indicating that the inhibitor incubation had no effect (Figure 2C). Then, we addressed whether the cytosolic antigen transfer required active transport machinery or whether it was a result of diffusion. This was tested via its dependency on ATP consumption during a temperature shift to 4°C. We found that in BMMs COX or PLA₂ inhibitor-induced transfer of β-lactamase to the cytosol required ATP because it did not occur at 4°C (Figure 2D). Together, we conclude that the effect of prostanoïds on the regulation of intracellular compartmentalization is specific for immune cells, particularly macrophage like cells, and it requires antigen internalization ruling out a potential involvement of non-specific plasma membrane damage in this process.

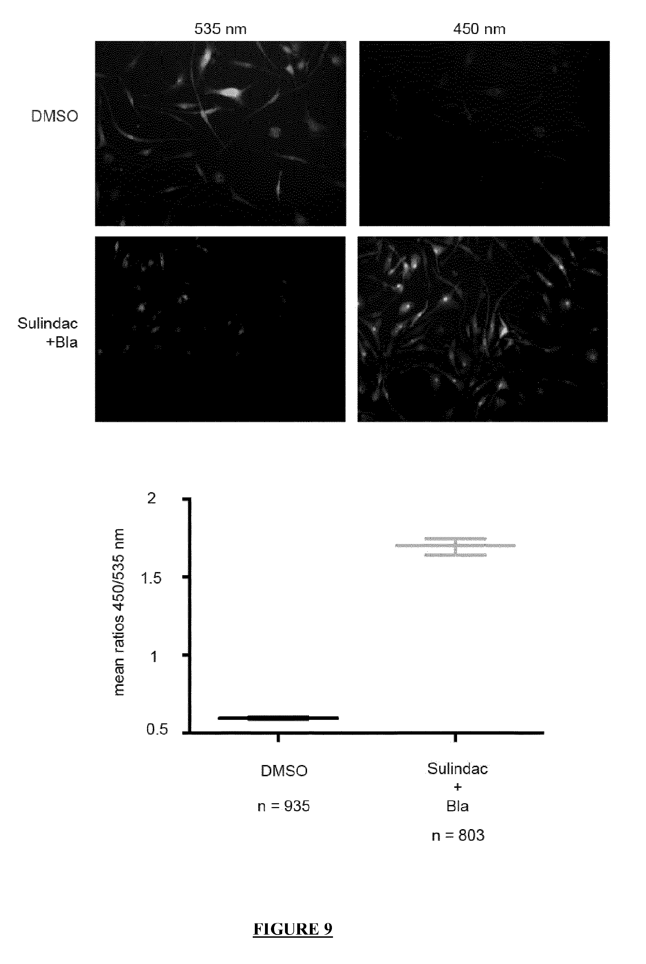

We decided to validate our results using an additional COX inhibitor, another independent experimental set-up, and other types of antigen (Figures 9, 10A-10C, 11 and 12A-12D). Incubating BMMs with the COX inhibitor Sulindac led to a robust transfer of β-lactamase to the cytosol as shown by the cleavage CCF4 following antigen loading (Figure 9). Furthermore, the extent of the effect was comparable to the one observed using indomethacin (Figure 2A). Another method commonly used to track cytosolic delivery of antigens makes use of fluorescently labeled dextran polymers. We decided to test the ability of BMMs to deliver fluorescent dextran to the cytosol. These sugar polymers are taken up by cells into vesicular compartments as highlighted in Figure 10A (upper panel). Strikingly, the staining switched to cytosolic staining in case BMMs were pretreated with indomethacin (Figure 10A) demonstrating cytoplasmic transfer of dextran from endomembrane compartments. It was further confirmed using other types of fluorescent antigens following indomethacin treatment. This highlighted that COX inhibition induced not only the transfer of Cy3-labelled β-lactamase (Figure 10B) but also Cy3 alone (Figure 10C) to the cytosol in BMMs. These observations confirmed the general importance of the cytosolic delivery boost of indomethacin in the context of different antigens including proteins as well as sugars and small molecules.

Moreover, using BV2 macrophage like cells, we measured that the effect of indomethacin is dose-dependent on the external concentration of β-lactamase. Indeed, increasing exogenous antigen concentration gradually raises cytosolic transfer (Figure 11). The same dose-dependency was observed when particulate antigens such as beads adsorbed with β-lactamase (Figures 12A and 12C) or bacteria associated (Figures 12B and 12D) β-lactamase were added to the macrophages instead of applying fluid-phase uptake.

In summary, we identified that the PLA₂/COX axis inhibition challenges endomembrane compartmentalization in macrophages. Importantly, this effect, although endocytosis-dependent, is not a consequence of increased antigen internalization, it is independent of plasma membrane damages and, it is specific for immune cells. This suggests that prostanoïd production from arachidonic acid constantly represses antigen transfer to the cytosol from endosomes. Inversely, interfering with prostanoïd biosynthesis leads to a robust cytosolic transfer.

### TXA₂ and arachidonic acid are key regulators of endosomal compartmentalization in macrophages

Since the cytosolic delivery of β-lactamase is most likely controlled by the levels of specific lipid mediators, we investigated which cellular constituents were involved. We focused on two candidates PGE2 and TXA₂. One experimental hurdle to monitor these mediators is their short half-life of less than one minute at 37°C. In order to circumvent this limitation, we performed complementation experiments using stable versions of them, dimethylPGE2 and U46619 (see materials and methods). After incubation with DMSO, indomethacin or AACOCF3, BMMs were complemented with either dimethylPGE2 or U46619 before β-lactamase challenge. Whereas dimethylPGE2 did not lead to a reversal of the effect of antigen transfer to the cytoplasm, U46619 reduced the inhibitor-induced antigen transfer to the cytosol (Figures 3A and 3B). This underlines the requirement of the lipid mediator TXA₂ as a potential negative regulator in the targeting of antigens to the cytosol. In order to confirm the involvement of this molecule, we decided to use ICI192605, an antagonist of the TXA₂ receptor (Figure 3C). Strikingly, a 15 minutes treatment with this compound led to a robust antigen transfer after 90 minutes of incubation with β-lactamase mimicking PLA2 and COX inhibition: this antagonist recapitulated the previously measured positive effect of cytosolic antigen transfer by AACOCF3 and indomethacin (Figure 3C). Nevertheless, since TXA₂ only partially complemented COX inhibition (Figures 3A and B), other molecules may also be implicated in the induced cytosolic transfer of antigen. Along these lines, we reasoned that the COX substrate arachidonic acid would be a potential candidate because it accumulates upon COX inhibition and is a precursor for a number of other lipid mediators like hydroxyeicosatetraenoic acids (HETEs), epoxytrienoic acids (EETs) and anandamide. Incubation with arachidonic acid for 15 minutes triggered cytosolic access of our model antigen β-lactamase as observed by blocking TXA₂ signaling (Figure 3C).

This highlights the implication of the PLA2/arachidonic acid/COX/TXA₂ axis in regulating cytosolic access of endosomal antigens in macrophages.

### Increased endosomal antigen stability is not sufficient for triggering cytosolic transfer in macrophages

We then investigated the molecular mechanism by which TXA₂ and arachidonic acid influence endosomal physiology regulating the permeability of this compartment. First, we tested if these lipid mediators target endosomal acidification processes through vacuolar type H+ adenosine triphosphatases (V-ATPases) and hydrolase activation. We therefore asked whether increasing antigen stability within endosomes would boost cytosolic transfer. To achieve this, we used Concanamycin B (conB), an inhibitor of endosomal acidification, and a of protease inhibitor cocktail (PI) in conjunction of our antigen transfer assay. As shown in figure 4, neither conB nor PI incubation led to any cytosolic transfer of β-lactamase. Even though these experiments cannot rule out an effect of lipid mediators on the intracellular antigen stability, they strongly suggested that additional subcellular pathways would be required for cytosolic transfer of antigens through an active process.

### Ubiquitination, phosphoinositol-biphosphate formation and HSP90 participate in the transfer of β-lactamase triggered by COX inhibition in macrophages

Our results suggested similarities between indomethacin induced cytosolic transfer of antigens in macrophages and well-characterized processes observed during cross-presentation in dendritic cells. Therefore, we decided to confront our findings with results from other research groups. One proposed mechanism involves the ubiquitination machinery and the molecular chaperone HSP90. Additionally, endosomal sorting after uptake through phospho-inositol modifications has been shown to enhance cytosolic delivery. We selected inhibitors from each of these different pathways in order to challenge the indomethacin triggered cytosolic transfer. Ubiquitination inhibiton was achieved adding Pyr41, HSP90 inhibition via radicicol, and endosomal sorting via the PI3K inhibitor wortmannin (Figures 4A-4B). Cytosolic antigen access was pulsed with a 15 minutes indomethacin treatment, and then BMMs were incubated with β-lactamase in the presence of the inhibitors for 90 minutes. We found that all three inhibitors with known effects in dendritic cells exhibited a similar behavior in the context of cytoplasmic transfer in macrophage-like cells (Figures 4A-4B). Particularly, we decreased cytosolic antigen transfer induced by indomethacin through ubiquitination inhibition via Pyr41.

From these experiments, we conclude that the newly identified mechanism of prostanoïd involvement in antigen trafficking towards the cytoplasm is relevant for macrophages and for dendritic cells

### COX inhibition promotes cross-presentation of ovalbumin in dendritic cells

Ubiquitination, endosomal sorting and HSP90 were shown not only to participate in the cytosolic transfer but also in the downstream events such as the presentation of exogenous antigens to T cells. To measure the consequence of increased cytoplasmic transfer of antigens through the prostanoid pathway, we decided to investigate the effect of indomethacin on cross-presentation in dendritic cells as most relevant involved cell type. Thereforen, BMDCs were treated for 15 minutes in the presence of DMSO, lactacystin or indomethacin, incubated with serial dilutions of ovalbumin for 4 hours and subjected to the B3Z *in vitro* cross-presentation assay (Figure 5). As expected, ovalbumin is cross-presented in a dose-dependent manner in DMSO treated BMDCs. However, BMDCs treated with lactacystin do not cross-present ovalbumin, even for very high extracellular concentration of antigen, thus corroborating our findings of the requirement for cytosolic transfer and proteasomal processing. In contrast, indomethacin incubated BMDCs displayed enhanced capacities for cross-priming compared to the DMSO control. Thus, beside its effect on the transfer of our model antigen β-lactamase from endosomes to the cytosol, indomethacin is also able to boost cross-presentation of ovalbumin, the most famous model antigen in immunology.

### DISCUSSION

We have discovered a subcellular pathway that leads to increased targeting to the cytosol of extracellular antigens through a prostanoïd biosynthesis block enhancing cross-presentation. Induced cytosolic delivery correlates with TXA₂ reduction and arachidonic acid accumulation. The involvement of these lipid mediators in modulating such a crucial biological process provides a striking link between inflammation and cross-presentation. Our study opens new possibilities aiming at the modulation of immune responses in the context of a variety of pathologies or vaccination.

Our novel microscopic assay to monitor cytoplasmic transfer of external antigens overcomes important experimental hurdles of antigen trafficking, such as indirect measurements of compartmental markers in immunofluorescence or contaminations during cell fractionation. Our method does not require any biochemical treatment or cell disruption, and it displays unprecedented accuracy, temporal resolution and functions in living cells. With the CCF4 FRET substrate of our assay, as few as 50 molecules of β-lactamase can be detected within one cell. Furthermore, it can be miniaturized and standardized for high-throughput/high-content screening. We cannot exclude that cleaved fragments of β-lactamase are transferred in the cytosol losing their enzymatic activity to hydrolyze CCF4. This observation fits with studies showing that the model antigen ovalbumin is partially cleaved before reaching the cytosol. Since our tracking system requires the transfer into the cytosol of at least some of the full-length β-lactamase to achieve CCF4 cleavage, it particularly enables to monitor early cytosolic transfer of antigens.

Using the CCF4/β-lactamase assay, we show that murine DCs have the intrinsic ability to perform this task efficiently. This result is in accordance with the high cross-presenting capacities of DCs in vivo and highlights their natural capacity to transfer antigens from endosomes to the cytosol without the need for additional external stimuli. Other groups have underlined this difference between mouse DCs and macrophages in vitro, as in vivo. Very recently, the CCF4/β-lactamase assay confirmed this in human derived APCs. The fact that cytosolic transfer is not detected in macrophages using our method does not mean that they are devoid of this capacity in general. It rather suggests the requirement for other signals involved in the transfer process, their endosomal/phagosomal degradative properties, or the lower abundance of efficient translocation machinery compared to dendritic cells.

We discovered a striking implication of eicosanoïds in regulating the access of extracellular antigens to the cytosol. Blocking prostanoïd biosynthesis through COX or PLA2 inhibition using multiple inhibitors of different implicated enzymes unlocked the poor ability of macrophages to transfer uptaken antigens to the cytosol. Also, three different types of antigens different types of antigens (β-lactamase as protein, dextran as sugar polymer, and a small molecule/Cy3) were used. Together, this hints at a non-specific transport not limited to specific proteins, but accessible at least to sugars and small charged molecules. We highlight the role of TXA₂ and arachidonic acid as key lipid mediators modulating the cytosolic transfer of internalized antigens. As respectively substrate and downstream product of COX, it is not surprising that arachidonic acid and TXA₂ display opposing effects. Upon COX inhibition, the depletion of TXA₂ and the accumulation of arachidonic acid could lead to the transfer of endosomal content toward the cytosol. This view is supported by the facts that a TXA₂agonist can restrain the transfer induced by COX inhibition and that a TXA₂antagonist as well as arachidonic acid reproduced the transfer induced by COX inhibition. It is noteworthy that arachidonic acid could either have an effect on its own or it could be metabolized through the LOX or epoxygenase pathway to respectively produce leukotrienes or HETEs/EETs.

Prostanoïds have been already implicated in cross-presentation, and our data provides a novel mechanism at the cellular level for their role. One research group showed that overproduction of PGE₂ by tumor cells *in vivo* prevents both direct and cross-priming of anti-tumor CD8+ T cells through the inhibition of DCs maturation. Additionally, an increase in MDSC and Treg coupled to a decrease in CD8+ T cells infiltration upon PGE₂ action in the tumor microenvironment is well documented.

Here, we show that the over production of TXA₂ can be detrimental in the context of cancer progression.

The continuous synthesis and secretion of these lipid mediators by tumor cells but also by infiltrated APCs could create and support a local tolerant microenvironment allowing the cancer to subvert the adaptive immune response hiding from cytotoxic T cells. This hypothesis is supported by the fact that deletion of PLA₂, the most upstream enzyme of the prostanoïd pathway leads to decreased number of polyps in the gut of mice. Since adverse cardiac secondary effects have precluded usage of COX inhibitors for cancer treatment, TXA₂ may represent an attractive new target. Besides the known roles of improving CD8+ T cells and decreasing Treg/MDSC infiltration, as well as optimizing DCs maturation, it could also lead to increased subcellular trafficking and presentation of tumoral antigens in APCs.

Knowledge on the modulation of antigen trafficking inside APCs with a particular focus on their cytosolic access will open new strategies to treat a number of life-threatening pathologies including cancer, chronic inflammation and recurrent infections. Since vaccination against cancer or intracellular pathogens requires the targeting of tumor or microbial derived antigen in the cytosol of APCs for triggering immune protection and memory, understanding the orchestration of events leading to endomembrane crossing is of crucial importance.

## Claims

1. A thromboxane A2 (TXA₂) inhibitor for use as a stimulator or inducer of MHC Class I cross-presentation of at least one antigen in the treatment and/or prevention and/or palliation of a cancer or infectious disease in a mammal, wherein said at least one antigen is a tumor antigen of said cancer or is an antigen of the infectious agent of said infectious disease respectively.

2. The TXA₂ inhibitor for use according to claim 1, wherein said administration comprises the local administration of said TXA₂ inhibitor at the primary site of said cancer or infectious disease.

3. The TXA₂ inhibitor for use according to claim 1 or 2, wherein said TXA₂ inhibitor is administered in an amount sufficient to stimulate MHC Class I cross-presentation of said at least one antigen by APCs, which are contained in said mammal and which have infiltrated the primary site of said cancer or infectious disease.

4. An immunogenic preparation, which comprises
i. at least one antigen, and/or at least one Antigen Presenting Cell (APC), in the cytosol of which said at least one antigen is contained, and which further comprises
ii. at least one thromboxane A2 (TXA₂) inhibitor, and/or at least one APC, in the cytosol of which said at least one TXA₂ inhibitor is contained,
wherein said at least one antigen or APC of i. and said at least one TXA₂ inhibitor or APC of ii. are in combination for simultaneous, separate or sequential administration to a mammal in need of MHC Class I cross-presentation of said at least one antigen.

5. The immunogenic preparation of claim 4, or the TXA₂ inhibitor for use according to any one of claims 1-3, wherein said at least one TXA₂ inhibitor is a Non-Steroidal Anti-Inflammatory Drug (NSAID) or a pharmaceutically acceptable salt thereof, and wherein said NSAID is an antagonist of the TXA₂ receptor and/or an inhibitor of TXA₂ biosynthesis.

6. The immunogenic preparation of claim 5, or the TXA₂ inhibitor for use according to claim 5, wherein said NSAID is:
**ICI-192605** (4-(Z)-6-(2-o-Chlorophenyl-4-o-hydroxyphenyl-1,3-dioxan-cis-5-yl)hexenoic acid),
**SQ-29548** ([1S-[1α,2α(Z),3α,4α]]-7-[3-[[2-[(phenylamino)carbonyl]hydrazino]methyl]-7-oxabicyclo[2.2.1]hept-2-yl]-5-heptenoic acid),
**terutroban** (3-[(6R)-6-(4-chlorobenzenesulfonamido)-2-methyl-5,6,7,8-tetrahydronaphthalen-1-yl]propanoic acid),
**ifetroban** (3-[2-[[(1S,2R,3S)-3-[4-(pentylcarbamoyl)-1,3-oxazol-2-yl]-7-oxabicyclo[2.2.1]heptan-2-yl]methyl]phenyl]propanoic acid),
**seratrodast** (7-(3,5,6-Trimethyl-1,4-benzoquinon-2-yl)-7-phenylheptanoic acid),
**solutroban** (2-[4-[2-(benzenesulfonamido)ethyl]phenoxy]acetic acid),
**SQ-28668** ((Z)-7-[(1S,2R,3S,4R)-3-[(E)-3-hydroxy-4-phenylpent-1-enyl]-7-oxabicyclo[2.2.1]heptan-2-yl]hept-5-enoic acid),
**ONO-3708** ((Z)-7-[(1R,3S,4S,5S)-3-[[(2R)-2-cyclopentyl-2-hydroxyacetyl]amino]-6,6-dimethyl-4-bicyclo[3.1.1]heptanyl]hept-5-enoic acid),
**ramatroban** (3R-[[(4-fluorophenyl)sulfonyl]amino]-1,2,3,4-tetrahydro-9H-carbazole-9-propanoic acid),
**EP-045** ((Z)-7-[(1R,2R,3R,4R)-3-[(E)-(phenylcarbamoylhydrazinylidene)methyl]-2-bicyclo[2.2.1]heptanyl]hept-5-enoic acid),
**domitroban** ((Z)-7-[(1R,2S,3S,4S)-3-(benzenesulfonamido)-2-bicyclo[2.2.1]heptanyl]hept-5-enoic acid),
**OKY-046** (3-[4-(1H-imidazol-1-ylmethyl)phenyl]-2E-propenoic acid), or
**picotamide** (4-methoxy-1-N,3-N-bis(pyridin-3-ylmethyl)benzene-1,3-dicarboxamide), or
a pharmaceutically acceptable salt thereof.

7. The immunogenic preparation of any one of claims 4-6, or the TXA₂ inhibitor for use according to any one of claims 1-3 and 5-6, wherein said TXA₂ inhibitor is:
- an antagonist of the TXA₂ receptor, and/or
- an inhibitor of TXA₂ biosynthesis,
which does not inhibit leukotriene biosynthesis.

8. The immunogenic preparation of any one of claims 4-7, or the TXA₂ inhibitor for use according to any one of claims 1-3 and 5-7, wherein said TXA₂ inhibitor is:
- an antagonist of the TXA₂ receptor, and/or
- an inhibitor of TXA₂ biosynthesis,
which does not inhibit prostaglandin E₂ (PGE₂) biosynthesis.

9. The immunogenic preparation of any one of claims 4-8, or the TXA₂ inhibitor for use according to any one of claims 1-3 and 5-8, wherein said inhibitor of TXA₂ biosynthesis is an inhibitor of TXA₂ synthase.

10. The immunogenic preparation of any one of claims 5-9, or the TXA₂ inhibitor for use according to any one of claims 5-9, wherein:
- said antagonist of the TXA₂ receptor has an EC50 for said TXA₂ receptor, which is lower than its EC50 for at least the PGE₂ receptor, and/or
- said inhibitor of TXA₂ biosynthesis has an EC50 for TXA₂ synthase, which is lower than its EC50 for PGE₂ synthase as well as lower than its EC50 for prostaglandin F₂ (PGF₂) synthase, prostaglandin D₂ (PGD₂) synthase and prostaglandin I₂ (PGI₂) synthase.

11. The immunogenic preparation of any one of claims 5-10, or the TXA₂ inhibitor for use according to any one of claims 5-10, wherein:
- said antagonist of the TXA₂ receptor specifically inhibits the TXA₂ receptor, and/or
- said inhibitor of TXA₂ biosynthesis specifically inhibits TXA₂ synthase.

12. The immunogenic preparation of any one of claims 4-11, or the TXA₂ inhibitor for use according to any one of claims 1-3 and 5-11, wherein said at least one antigen is tumor antigen, or an antigen of an infectious agent.

13. The immunogenic preparation of any one of claims 4-12, or the TXA₂ inhibitor for use according to any one of claims 1-3 and 5-12, wherein said APC(s) is(are) macrophage(s).

14. The immunogenic preparation of any one of claims 4-13, which is for use in the immuno-prevention, immuno-therapy or immuno-palliation of a cancer or of an infectious disease.

15. The immunogenic preparation of claim 14, or the TXA₂ inhibitor for use according to any one of claims 1-3 and 5-13, wherein said cancer is not a cancer of the Central Nervous System.

16. The immunogenic preparation of claim 14 or 15, or the TXA₂ inhibitor for use according to any one of claims 1-3, 5-13 and 15, wherein said cancer is not a COX2-overpressing cancer.

17. The immunogenic preparation of any one of claims 4-16, or the TXA₂ inhibitor for use according to any one of claims 1-3, 5-13 and 15-16, which is for use to induce or stimulate the priming of Cytotoxic T Lymphocytes by APC of said mammal.

18. The immunogenic preparation of any one of claims 4-17, or the TXA₂ inhibitor for use according to any one of claims 1-3, 5-13 and 15-17, which is for use in patients, who have a defect in MHC class I cross-presentation.


SEQUENCE LISTING

<110>  INSTITUT PASTEUR

<120>  TXA2 inhibitor as stimulator or inducer of MHC Class I
       cross-presentation

<130>  B10797 - FP/BA

<160>  9

<170>  PatentIn version 3.5

<210>  1
<211>  2619
<212>  DNA
<213>  Homo sapiens

<400>  1
cccacccgtc ccagctcggc ttagcccccg cggacccctc caggccgcga ccccagggcg      60

tcctgtgccg ccacgccctc catctgtgtg ggtcctctgc tgggcccgcc cctggtcaca     120

gccagactga ctcagtttcc ctgggaggtc ccgctcgagc ccgtccttcc cctccctctg     180

cccgccccca gccctcgccc caccctcggc gcccgcacat ctgcctgctc agctccagac     240

ggcgcccgga cccccgggcg cgggatccag ccaggtggga gccccgcaga tgaggtctct     300

gaaggtgtgc ctgaaccagt gccagcctgc cctgtctgca gcatcggcct gatggggtgg     360

tgactgatcc ctcagggctc cggagccatg tggcccaacg gcagttccct ggggccctgt     420

ttccggccca caaacattac cctggaggag agacggctga tcgcctcgcc ctggttcgcc     480

gcctccttct gcgtggtggg cctggcctcc aacctgctgg ccctgagcgt gctggcgggc     540

gcgcggcagg ggggttcgca cacgcgctcc tccttcctca ccttcctctg cggcctcgtc     600

ctcaccgact tcctggggct gctggtgacc ggtaccatcg tggtgtccca gcacgccgcg     660

ctcttcgagt ggcacgccgt ggaccctggc tgccgtctct gtcgcttcat gggcgtcgtc     720

atgatcttct tcggcctgtc cccgctgctg ctgggggccg ccatggcctc agagcgctac     780

ctgggtatca cccggccctt ctcgcgcccg gcggtcgcct cgcagcgccg cgcctgggcc     840

accgtggggc tggtgtgggc ggccgcgctg gcgctgggcc tgctgcccct gctgggcgtg     900

ggtcgctaca ccgtgcaata cccggggtcc tggtgcttcc tgacgctggg cgccgagtcc     960

ggggacgtgg ccttcgggct gctcttctcc atgctgggcg gcctctcggt cgggctgtcc    1020

ttcctgctga acacggtcag cgtggccacc ctgtgccacg tctaccacgg gcaggaggcg    1080

gcccagcagc gtccccggga ctccgaggtg gagatgatgg ctcagctcct ggggatcatg    1140

gtggtggcca gcgtgtgttg gctgcccctt ctggtcttca tcgcccagac agtgctgcga    1200

aacccgcctg ccatgagccc cgccgggcag ctgtcccgca ccacggagaa ggagctgctc    1260

atctacttgc gcgtggccac ctggaaccag atcctggacc cctgggtgta tatcctgttc    1320

cgccgcgccg tgctccggcg tctccagcct cgcctcagca cccggcccag gtcgctgtcc 1380

ctccagcccc agctcacgca gcgctccggg ctgcagtagg aagtggacag agcgcccctc 1440

ccgcgccttt ccgcggagcc cttggcccct cggacagccc atctgcctgt tctgaggatt 1500

caggggctgg gggtgctgga tggacagtgg gcatcagcag cagggttttg ggttgacccc 1560

aatccaaccc ggggaccccc aactcctccc tgatcctttt accaagcact ctcccttcct 1620

cggccccttt ttcccatcca gagctcccac cccttctctg cgtccctccc aaccccagga 1680

agggcatgca gacattggaa gagggtcttg cattgctatt ttttttttta gacggagtct 1740

tgctctgtcc cccaggctgg agtgcagtgg cgcaatctca gctcactgca acctccacct 1800

cccgggttca agcgattctc ctgcctcagc ctcctgagta gctgggacta taggcgcgcg 1860

ccaccacgcc cggctaattt ttgtattttt agtagagacg gggtttcacc gtgttggcca 1920

ggctggtctt gaactcctga cctcaggtga ttcaccagcc tcagcctccc aaagtgctgg 1980

gatcacaggc atgaaccacc acacctggcc attttttttt ttttttttag acggagtctc 2040

actctgtggc ccagcctgga gtacagtggc acgatctcgg ctcactgcaa cctccgcctc 2100

ccgggttcaa gcgattctcg tgcctcagcc tcccgagcag ctgggattac aggcgtaagc 2160

cactgcgccc ggccttgcat gctctttgac cctgaatttg acctacttgc tggggtacag 2220

ttgcttcctt ttgaacctcc aacagggaag gctctgtcca gaaaggattg aatgtgaacg 2280

ggggcacccc cttttcttgc caaaatatat ctctgccttt ggttttattt tcctttgggt 2340

ccagaagttt tcaatctctg gagtttgtgc attgggcctc acccggagca gacgagggca 2400

gggctgggaa ggatggggag ggacatagag gattcggttc ctcccccacc ttgtttcttg 2460

agtgtttcct aatcacttga tgggtggtac cacctagaat tcttccagca gcctgtaatg 2520

ggagggctac aggttagccc tggaacctgc aatcaaacca cctttgaatc tgtgtgtcat 2580

taaaagtaga tataaatggg caaaaaaaaa aaaaaaaaa 2619

<210> 2
<211> 343
<212> PRT
<213> Homo sapiens

<400> 2

<210>  3
<211>  407
<212>  PRT
<213>  Homo sapiens

<400>  3

Met Trp Pro Asn Gly Ser Ser Leu Gly Pro Cys Phe Arg Pro Thr Asn
1               5                   10                  15

Ile Thr Leu Glu Glu Arg Arg Leu Ile Ala Ser Pro Trp Phe Ala Ala
            20                  25                  30

Ser Phe Cys Val Val Gly Leu Ala Ser Asn Leu Leu Ala Leu Ser Val
        35                  40                  45

Leu Ala Gly Ala Arg Gln Gly Gly Ser His Thr Arg Ser Ser Phe Leu
    50                  55                  60

Thr Phe Leu Cys Gly Leu Val Leu Thr Asp Phe Leu Gly Leu Leu Val
65                  70                  75                  80

Thr Gly Thr Ile Val Val Ser Gln His Ala Ala Leu Phe Glu Trp His
                85                  90                  95

Ala Val Asp Pro Gly Cys Arg Leu Cys Arg Phe Met Gly Val Val Met
            100                 105                 110

Ile Phe Phe Gly Leu Ser Pro Leu Leu Leu Gly Ala Ala Met Ala Ser
        115                 120                 125

Glu Arg Tyr Leu Gly Ile Thr Arg Pro Phe Ser Arg Pro Ala Val Ala
    130                 135                 140

Ser Gln Arg Arg Ala Trp Ala Thr Val Gly Leu Val Trp Ala Ala Ala
145                 150                 155                 160

Leu Ala Leu Gly Leu Leu Pro Leu Leu Gly Val Gly Arg Tyr Thr Val
                165                 170                 175

Gln Tyr Pro Gly Ser Trp Cys Phe Leu Thr Leu Gly Ala Glu Ser Gly
            180                 185                 190

Asp Val Ala Phe Gly Leu Leu Phe Ser Met Leu Gly Gly Leu Ser Val
        195                 200                 205

Gly Leu Ser Phe Leu Leu Asn Thr Val Ser Val Ala Thr Leu Cys His
    210                 215                 220

Val Tyr His Gly Gln Glu Ala Ala Gln Gln Arg Pro Arg Asp Ser Glu
225                 230                 235                 240

Val Glu Met Met Ala Gln Leu Leu Gly Ile Met Val Val Ala Ser Val
                245                 250                 255

Cys Trp Leu Pro Leu Leu Val Phe Ile Ala Gln Thr Val Leu Arg Asn
            260                 265                 270

Pro Pro Ala Met Ser Pro Ala Gly Gln Leu Ser Arg Thr Thr Glu Lys
        275                 280                 285

Glu Leu Leu Ile Tyr Leu Arg Val Ala Thr Trp Asn Gln Ile Leu Asp
    290                 295                 300

Pro Trp Val Tyr Ile Leu Phe Arg Arg Ala Val Leu Arg Arg Leu Gln
305                 310                 315                 320

Pro Arg Leu Ser Thr Arg Pro Arg Arg Ser Leu Thr Leu Trp Pro Ser
                325                 330                 335

Leu Glu Tyr Ser Gly Thr Ile Ser Ala His Cys Asn Leu Arg Leu Pro
            340                 345                 350

Gly Ser Ser Asp Ser Arg Ala Ser Ala Ser Arg Ala Ala Gly Ile Thr
        355                 360                 365

Gly Val Ser His Cys Ala Arg Pro Cys Met Leu Phe Asp Pro Glu Phe
    370                 375                 380

Asp Leu Leu Ala Gly Val Gln Leu Leu Pro Phe Glu Pro Pro Thr Gly
385                 390                 395                 400

Lys Ala Leu Ser Arg Lys Asp
                405

<210>  4

<211>  10
<212>  PRT
<213>  Homo sapiens

<400>  4

Phe Ile Ala Ser Asn Gly Val Lys Leu Val
1               5                   10


<210>  5
<211>  9
<212>  PRT
<213>  Homo sapiens

<400>  5

Glu Thr Val Ser Glu Gln Ser Asn Val
1               5


<210>  6
<211>  8
<212>  PRT
<213>  Homo sapiens

<400>  6

Gln Gln Ile Thr Lys Thr Glu Val
1               5


<210>  7
<211>  9
<212>  PRT
<213>  Homo sapiens

<400>  7

Phe Leu Asp Glu Phe Met Glu Gly Val
1               5


<210>  8
<211>  9
<212>  PRT
<213>  Homo sapiens

<400>  8

Phe Pro Ser Asp Ser Trp Cys Tyr Phe
1               5


<210>  9
<211>  9
<212>  PRT
<213>  Homo sapiens

<400>  9

Val Val Pro Cys Glu Pro Pro Glu Val
1               5